(19) Europäisches Patentamt
European Patent Office
Office européen des brevets

(11) Publication number: **0 527 427 B1**

# (12) EUROPEAN PATENT SPECIFICATION

(45) Date of publication of patent specification: **15.11.95**

(51) Int. Cl.[6]: **C08G 18/18**, C08G 18/20, C08G 18/10, A61L 15/07

(21) Application number: **92113324.5**

(22) Date of filing: **05.08.92**

(54) **Catalysts and casting article, method, and composition.**

(30) Priority: **08.08.91 US 742048**
**08.08.91 US 742047**

(43) Date of publication of application:
**17.02.93 Bulletin 93/07**

(45) Publication of the grant of the patent:
**15.11.95 Bulletin 95/46**

(84) Designated Contracting States:
**DE FR GB IT**

(56) References cited:
**EP-A- 0 407 056**
**DE-A- 3 134 592**
**FR-A- 2 207 949**

**PATENT ABSTRACTS OF JAPAN 28 June 1991 & JP-30 84 021**

(73) Proprietor: **MINNESOTA MINING AND MANUFACTURING COMPANY**
**3M Center,**
**P.O. Box 33427**
**St. Paul,**
**Minnesota 55133-3427 (US)**

(72) Inventor: **Scholz, Matthew T., c/o Minnesota Mining and**
**Manufact. Co.,**
**2501 Hudson Road,**
**P.O. Box 33427**
**St. Paul,**
**Minnesota 55133-3427 (US)**
Inventor: **Scherrer, Robert A., c/o Minnesota Mining and**
**Manufact. Co.,**
**2501 Hudson Road,**
**P.O. Box 33427**
**St. Paul,**
**Minnesota 55133-3427 (US)**

(74) Representative: **Werner, Hans-Karsten, Dr. et al**
**Patentanwälte**
**Von Kreisler-Selting-Werner**
**Postfach 10 22 41**
**D-50462 Köln (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

### Field of the Invention

This invention relates to catalysts for curing isocyanate-functional materials. In another aspect, this invention relates to curable compositions comprising an isocyanate-functional material and a catalyst. This invention also relates to casting articles and methods of orthopedic casting.

### Background of the Invention

Orthopedic casts for use in treating bone fractures or other conditions requiring immobilization of a body member are generally formed from a sheet of fabric or scrim material coated or impregnated with a substance that hardens into a rigid structure after the sheet has been wrapped around the body member.

Many orthopedic casts now commonly used are comprised of a backing impregnated with a water-curable isocyanate-functional prepolymer. The backing can be knitted, woven, or nonwoven scrim comprised of natural, polymeric, or glass fibers. The preferred scrim materials are knitted fiberglass scrims. These casts when cured have a higher strength to weight ratio than plaster-of-paris, are more resistant to water and provide good radiolucency.

U. S. Pat. No. 4,411,262 (von Bonin), U. S. Pat. No. 4,502,479 (Garwood), and U.S. Pat. No. 4,667,661 (Scholz et al.) disclose water-curable isocyanate-functional prepolymers useful in orthopedic bandages. The prepolymer typically includes a tertiary amine catalyst in an amount selected to optimize the "set" time. After the resin-impregnated scrim has been immersed in water, sufficient "working time", e.g., 3 to 5 minutes, should be provided in which the wrapping is accomplished and the cast is manually molded into a desired shape. However, after the cast is shaped, the resin should continue to harden and rapidly build strength, typically in 15-30 minutes, into a rigid, high-strength, weight-bearing cast.

U. S. Pat. No. 4,376,438 (Straube et al.) discloses an orthopedic casting material wherein the tertiary amine catalyst is incorporated into the backbone of the polymer portion of the isocyanate-functional prepolymer. No separate catalyst is required.

U. S. Pat. No. 4,502,479 (Garwood et al.) discloses the use of tertiary alkanolamines, e.g., dimethylethanolamine, as catalysts in the curing of a water-curable isocyanate-functional prepolymer. At concentrations which do not adversely affect shelf stability, these catalysts do not cure as fast as desired by many experienced cast appliers.

U. S. Pat. No. 4,433,680 (Yoon) discloses the use of 2,2'-dimorpholinyldiethyl ether (DMDEE) as a catalyst in the cure of a water-curable isocyanate-functional prepolymer on an open-weave fibrous substrate to form an orthopedic bandage.

U. S. Pat. No. 4,705,840 (Buckanin) discloses the use of 2,2'-dimorpholinyldialkyl ethers substituted on one of the carbon atoms alpha to the central ether oxygen atom as catalysts in the curing of water-curable isocyanate-functional prepolymers.

Various compounds of Formula I below are known. For example, 2-(dialkylamino)ethyl 1-alkylprolinates are described in West German Pat. No. DE 1,933,411 (Likhosherstov et al., abstracted at Chemical Abstracts 76:153583m), Khim.-Farm.Zh. 1967, 1, 26 (Lebedeva et al., abstracted at Chemical Abstracts 67:82022d), and Khim.-Farm.Zh. 1973, 7, 10 (Likhosherstov et al., abstracted at Chemical Abstracts 79:53122d) as low toxicity ganglioplegic agents. Similarly, 2-(dialkylamino)ethyl 1-methylpipecolinates are described in Soviet Union Pat. No. 278027 (Likhosherstov et al., abstracted at Chemical Abstracts 74:53512c) as pharmaceutical agents. 2-(1-Piperidinyl)ethyl and 2-(4-morpholinyl)ethyl 4-morpholineacetates and 1-piperidineacetates are described in Acta. Pol. Pharm. 1979, 36, 1 (Wolinski et al., abstracted at Chemical abstracts 91:123691n) as anticholinergic agents, and various aminoalkyl esters of piperidino- and morpholino-acetic acid are described in Acta. Pol. Pharm. 1980, 37, 397 (Wolinski et al., abstracted at Chemical Abstracts 95:80069d) and Pol. J. Pharmacol. Pharm. 1978, 30, 497 (Faff et al., abstracted at Chemical Abstracts 91:32648n). 2-(Diethylamino)ethyl dimethylaminoacetate is described in Farm. Zh. - (Kiev) 1990, 1, 38 (Grinevich et al., abstracted at Chemical Abstracts 114:42025q) as having significant inotropic activity. Also, 2-(diethylamino)ethyl N,N-dimethylglycinate is described in Khim.-Farm.Zh. 1983, 17, 916 (Razina et al., abstracted at Chemical Abstracts 99:169415u) and said to possess analgesic activity.

FR-A-2 207 949 recommends to replace bad smelling morpholine catalysts with carboxylic ester catalysts where both the acid and the ester part are substituted with a tertiary amino group.

Summary of the Invention

This invention provides curable compositions comprising an isocyanate-functional prepolymer and a catalytically effective amount of a compound of Formula I

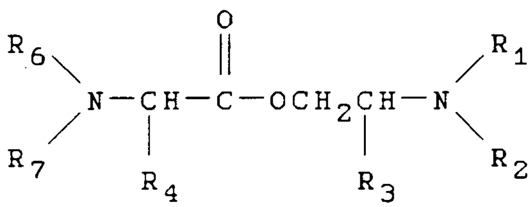

I

wherein:

$R_1$ and $R_2$ are independently straight chain, branched chain, or cyclic alkyl of one to six carbon atoms, or $R_1$ and $R_2$ together form a straight chain or branched chain alkylene group having four or five carbons in the main alkylene chain, or $R_1$ and $R_2$ together form a group of the formula

-A-O-B- or

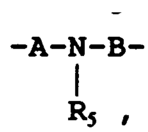

wherein A and B are independently straight chain or branched chain alkylene groups each having two carbon atoms in their main alkylene chain and $R_5$ is alkyl, aryl, or a deactivating substituent;

$R_3$ is hydrogen or straight chain, branched chain, or cyclic alkyl of one to six carbon atoms, or $R_1$ and $R_3$ together form a straight chain or branched chain alkylene group having three or four carbon atoms in the main alkylene chain, with the proviso that when $R_1$ and $R_3$ together form a straight chain or branched chain alkylene group having three or four carbon atoms in the main alkylene chain, then $R_2$ is straight chain, branched chain, or cyclic alkyl of one to about six carbon atoms;

$R_4$ is hydrogen, straight chain, branched chain, or cyclic alkyl of one to six carbon atoms, $R_6$ and $R_7$ are independently straight chain, branched chain, or cyclic alkyl of one to six carbon atoms, or $R_6$ and $R_7$ together form a straight chain or branched chain alkylene or alkenylene group with four or five carbons in the main alkylene or alkenylene chain, or $R_6$ and $R_7$ together form a group of the formula

-A-O-B- or

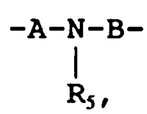

wherein A, B, and $R_5$ are as defined hereinabove, or $R_4$ and $R_6$ together form a straight chain or branched chain alkylene group having three or four carbon atoms in the main alkylene chain, with the proviso that when $R_4$ and $R_6$ together form a straight chain or branched chain alkylene group having three or four carbon atoms in the main alkylene chain, then $R_7$ is straight chain, branched chain, or cyclic alkyl of one to six carbon atoms.

In addition, this invention provides casting articles comprising a flexible sheet with a coating of the above-described curable composition thereon.

This invention also provides a catalysis method for catalyzing the cure of a water-curable isocyanate-functional prepolymer comprising forming a mixture of:

a) an isocyanate-functional material,

b) water, and

c) a catalytically effective amount of a compound of Formula I.

Also, this invention provides methods of orthopedic casting using the above-described casting articles.

The use of the compounds of Formula I as catalysts affords water-curable compositions having good set times, satisfactory shelf stability and, when curing is initiated, affords materials having surprisingly good early strengths when compared to compositions comprising commonly used catalysts of the prior art. The compositions of the invention are useful as adhesives, foams, coatings, and sealants, and as the curable component of an orthopedic bandage.

This invention further provides compounds of Formula II:

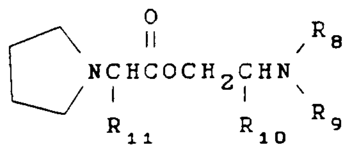

II

wherein:

$R_8$, and $R_9$ are independently straight chain, branched chain, or cyclic alkyl of one to six carbon atoms, or $R_8$ and $R_9$ together form a straight chain or branched chain alkylene group having four or five carbons in the main alkylene chain, or $R_8$ and $R_9$ together form a group of the formula -A-O-B- or

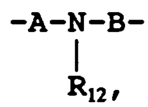

wherein A and B are independently straight chain or branched chain alkylene groups each having two carbon atoms in their main alkylene chain and $R_{12}$ is alkyl, aryl, or a deactivating substituent;

$R_{10}$ is hydrogen or straight chain, branched chain, or cyclic alkyl of one to six carbon atoms, or $R_8$ and $R_{10}$ together form a straight chain or branched chain alkylene group having three or four carbon atoms in the main alkylene chain, with the proviso that when $R_8$ and $R_{10}$ together form a straight chain or branched chain alkylene group having three or four carbon atoms in the main alkylene chain, then $R_9$ is straight chain, branched chain, or cyclic alkyl of one to six carbon atoms; and $R_{11}$ is hydrogen, straight chain, branched chain, or cyclic alkyl of one to six carbon atoms.

This invention also provides coating articles, catalysis methods, and methods of orthopedic casting involving compounds of Formula II.

## Detailed Description of the Invention

The compounds suitable for use as catalysts in this invention include compounds of Formulas I and II above. In Formula I $R_1$ and $R_2$ can be independently straight chain, branched chain, or cyclic alkyl of one to six carbon atoms. $R_3$ can be hydrogen or straight chain, branched chain, or cyclic alkyl of one to six carbon atoms. Alternatively, $R_1$ and $R_2$ along with the catenary nitrogen therebetween can form a pyrrolidine ring or a piperidine ring.

When $R_1$ and $R_2$ together form a group of the formula -A-O-B- or

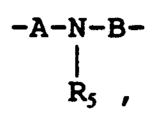

a morpholine ring or an N'-substituted piperazine ring for example can be formed. The N'-substituent $R_5$ is preferably a deactivating substituent, i.e., a group that substantially reduces the basicity of the N' nitrogen. For example alkylcarbonyl, arylcarbonyl, alkoxycarbonyl [-C(O)Oalkyl], dialkylaminocarbonyl [-C(O)N(alkyl)-$_2$], alkylaminocarbonyl, arylaminocarbonyl, alkylarylaminocarbonyl, and the like are suitable and others can be easily selected by those skilled in the art. $R_5$ can also be an aryl group such as phenyl, naphthyl, or the like, including substituted aryl such as methylphenyl (i.e., tolyl) or methylnaphthyl. When $R_5$ is alkyl the number of carbon atoms in the alkyl group is not unduly critical to the utility of the compound as a catalyst.

As a further alternative, $R_1$ and $R_3$ together can form a straight chain or branched chain alkylene chain having three or four carbons in the main alkylene chain, i.e., $R_1$ and $R_3$ along with the catenary nitrogen and the methine carbon therebetween can form a pyrrolidine or piperidine ring. In an instance wherein $R_1$ and $R_3$ form a pyrrolidine or piperidine ring, $R_2$ is straight chain, branched chain, or cyclic alkyl of one to six carbon atoms (i.e., the ring is an N-alkyl pyrrolidine or piperidine ring). $R_4$ and $R_6$ can optionally form a piperidine ring, in which case $R_7$ is straight chain, branched chain, or cyclic alkyl of one to six carbon atoms (i.e., the compound is an ester of an N-alkyl-2-piperidine carboxylic acid, sometimes referred to as a 1-alkylpipecolinate). $R_4$ and $R_6$ can also optionally form a pyrrolidine ring in which case also $R_7$ is straight chain, branched chain, or cyclic alkyl of one to six carbon atoms (i.e., the compound is an ester of an N-alkyl proline).

Some of the preferred compounds of Formula I include: 2-(N,N-dimethylamino)ethyl 1-methylpipecolinate; 2-(1-methylpiperidyl)methyl 1-methylpipecolinate; 2-(1-piperidino)ethyl 1-piperidinoacetate; 2-(1-methylpiperidyl)methyl N,N-dimethylaminoacetate; 2-(1-morpholino)ethyl N,N-diethylaminoacetate; 2-(1-morpholino)ethyl alpha-(N-morpholino)propionate; 2-(N,N-dimethylamino)ethyl N,N-dimethylaminoacetate; and 2-(1-piperidino)ethyl N,N-dimethylaminoacetate; and 2-(1-pyrrolidino)ethyl 1-methylpipecolinate. Most preferred are 2-(1-methylpiperidyl)methyl 1-methylpipecolinate and 2-(1-pyrrolidino)ethyl 1-methylpipecolinate.

These compounds are preferred because, when combined with an isocyanate-functional prepolymer, they provide resins with superior strength soon after curing is initiated when compared with the commonly used catalysts of the prior art.

As for Formula II, $R_8$ and $R_9$ can be independently straight chain, branched chain, or cyclic alkyl of one to six carbon atoms. $R_{10}$ can be hydrogen or straight chain, branched chain, or cyclic alkyl of one to six carbon atoms. Alternatively, $R_8$ and $R_9$ along with the catenary nitrogen therebetween can form a pyrrolidine ring or a piperidine ring.

When $R_8$ and $R_9$ together form a group of the formula -A-O-B- or

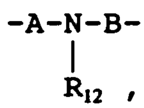

a morpholine ring or an N'-substituted piperazine ring for example can be formed. The N'-substituent $R_{12}$ is preferably a deactivating substituent, i.e., a group that substantially reduces the basicity of the N' nitrogen. For example, alkylcarbonyl, arylcarbonyl, alkoxycarbonyl [-C(O)Oalkyl], dialkylaminocarbonyl [-C(O)N(alkyl)-$_2$], alkylaminocarbonyl, arylaminocarbonyl, alkylarylaminocarbonyl, and the like are suitable and others are easily selected by those skilled in the art. $R_{12}$ can also be an aryl group such as phenyl, naphthyl, and the like, including substituted aryl such as methylphenyl (i.e., tolyl) and methylnaphthyl. When $R_{12}$ is alkyl the number of carbons in the alkyl group is not unduly critical to the utility of the compounds as catalysts.

As a further alternative, $R_8$ and $R_{10}$ together can form a straight chain or branched chain alkylene chain having three or four carbons in the main alkylene chain, i.e., $R_8$ and $R_{10}$ along with the catenary nitrogen and the methine carbon therebetween can form a pyrrolidine or piperidine ring. In an instance wherein $R_8$ and $R_{10}$ form a pyrrolidine or piperidine ring, $R_9$ is straight chain, branched chain, or cyclic alkyl of one to six carbon atoms (i.e., the ring is an N-alkyl pyrrolidine or piperidine ring).

Preferred compounds of Formula II include: 2-(1-piperidino)ethyl 1-pyrrolidineacetate; 2-(4-morpholino)-ethyl 1-pyrrolidineacetate; 2-(N,N-diethylamino)ethyl 1-pyrrolidineacetate; 2-(N,N-dimethylamino)ethyl 1-pyrrolidineacetate; and 2-(1-methylpiperidyl)methyl 1-pyrrolidineacetate. Most preferred is 2-(1-pyrrolidino)ethyl 1-pyrrolidineacetate. These compounds too are preferred because, when combined with an isocyanate-functional prepolymer, they provide resins with superior strength soon after curing is initiated when compared with the commonly used catalysts of the prior art.

Compounds of Formula I contain a nitrogen both in the alcohol residue of the ester and in the acid residue of the ester. They can be prepared by the transesterification reaction shown below, wherein the alkoxy portion of an ester of Formula III is replaced by the alkoxy portion of a compound of Formula IV:

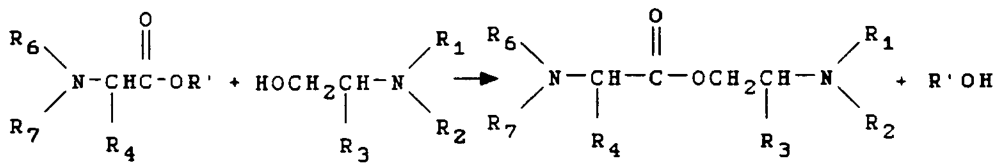

III IV I

and wherein $R_1$, $R_2$, $R_3$, $R_4$, $R_6$ and $R_7$ are as defined above, and "-OR'" designates an alkoxy, phenoxy, or other group capable of being displaced during the transesterification reaction.

The transesterification reaction can be carried out under conventional conditions, e.g., conditions involving such catalysts as dibutyl tin oxide, titanium isopropoxide, alkali metals, alkali metal hydrides and the like. Preferably the catalyst is sodium or potassium or the hydrides of these metals, because the residues of these catalysts are readily neutralized and separated from the product. Tin catalysts are less preferred, because the presence of tin compounds in an isocyanate-functional material can decrease the shelf stability of the material by catalyzing undesirable side reactions such as allophanate formation.

Many synthetic intermediates of Formulas III and IV are known. Others can be readily prepared from other known compounds by methods well known to those skilled in the art. Some compounds of Formula III, for example, are alkyl esters of either alpha-(N,N-dialkylamino)acetic acids or 1-cyclic amine substituted acetic acids. Such compounds can be prepared from alkyl α-haloalkanoates (wherein halo is bromo or chloro) by reaction with suitable secondary amines. 2-Carboxy-N-heterocyclics of Formula III can be prepared, e.g., by catalytic reduction of pyridine-2-carboxylic acids followed by methylation, or by catalytic reduction of an N-methyl quaternary salt of a 2-carboxypyridine.

Compounds of Formula IV are 2-(N,N-disubstituted amino)alkyl alcohols. These compounds are also generally known and can be prepared, for example, from the corresponding secondary amine and a suitable epoxide by methods well known to those skilled in the art.

The compounds of Formula II can be prepared by the transesterification reaction shown below, wherein the alkoxy portion of an ester of Formula V is replaced by the alkoxy portion of a compound of Formula VI:

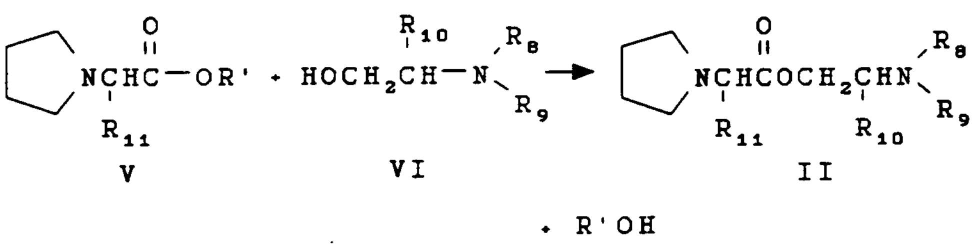

V VI II

and wherein $R_8$, $R_9$, $R_{10}$, and $R_{11}$ are as defined above, and "-OR'" designates an alkoxy, phenoxy, or other group capable of being displaced during the transesterification reaction.

The transesterification reaction can be carried out under conventional conditions, e.g., conditions involving such catalysts as dibutyl tin oxide, titanium isopropoxide, alkali metals, alkali metal hydrides and the like. Preferably the catalyst is sodium or potassium or the hydrides of these metals, because the residues of these catalysts are readily neutralized and separated from the product. Tin catalysts are less preferred, because the presence of tin compounds in an isocyanate-functional material can decrease the shelf stability of the material by catalyzing undesirable side reactions such as allophanate formation.

Synthetic intermediates of Formulas V and VI are known, or can be readily prepared from other known compounds by methods well know to those skilled in the art. Compounds of Formula V, for example, are alkyl esters of either pyrrolidineacetic acid or 1-substituted pyrrolidineacetic acids. Such compounds can be prepared as described in the Examples below from alkyl α-haloalkanoates (wherein halo is bromo or chloro) by reaction with pyrrolidine. Compounds of Formula VI are 2-(N,N-disubstituted amino)alkyl alcohols. These compounds are also generally known and can be prepared, for example, from the corresponding secondary amine and a suitable epoxide by methods well known to those skilled in the art.

Compounds of Formulas I and II are useful as catalysts for curing any isocyanate-functional material or composition. They are particularly useful for curing water-curable isocyanate-functional prepolymers. In this regard it is notable that the catalysts of the invention comprise an ester group and therefore would be expected to be relatively easily hydrolysed. Nonetheless, they function surprisingly well as catalysts of a reaction involving water.

Preferred prepolymers for use with the compounds of Formulas I and II are based on aromatic isocyanates. Such prepolymers are generally prepared by reacting a polyol with an excess of a polyisocyanate under conventional conditions. Such prepolymers are well known to those skilled in the art and are disclosed, e.g., in U.S. Pat. Nos. 4,411,262 (von Bonin et al.), 4,433,680 (Yoon), 4,502,479 (Garwood et al.), 4,667,661 (Scholz et al.), 4,705,840 (Buckanin), and 4,758,648 (Rizk et al.). A suitable prepolymer for use in the curable compositions of the invention uses an isocyanate known as ISONATE™ 2143L isocyanate (a mixture containing about 73% of diphenylmethane-4,4'-diisocyanate, Dow) and a polypropylene oxide polyol known as NIAX™ Polyol PPG 725 (AC West Virginia Polyol Co.). To prolong the shelf-life of the material, it is preferred to include about 0.02-0.5 percent by weight of benzoyl chloride or other suitable stabilizer. The most preferred curable compositions, casting articles, catalysis methods, and orthopedic casting methods of the invention involve prepolymers described in European Patent Application No. 90306696.7.

The curable compositions of the invention comprise an isocyanate-functional material and a catalytically effective amount of a compound of Formula I or Formula II. As used herein, the term "an effective amount" designates an amount of a component sufficient to provide the desired physical properties (e.g., cure rate, layer to layer lamination, and strength) to the curable composition. The particular amount of compound that constitutes a catalytically effective amount will vary with the particular compound used, the particular isocyanate-functional material used, the particular applications of the curable composition, and the set time that is desired for the curable composition. Particular amounts are easily selected by those skilled in the art and are set forth generally below with respect to particular applications.

In order to prepare a curable composition of the invention, an isocyanate-functional material and a compound of Formula I or Formula II can be mixed using conventional mixing techniques. In order to avoid premature curing of the resulting curable composition, the mixing should be done under anhydrous conditions, preferably in a substantially inert atmosphere, e.g., nitrogen gas. The resulting curable composition should also be stored under anhydrous conditions in a container substantially impermeable to oxygen and water vapor.

The curable compositions of the invention can be cured by exposure to water, e.g., water vapor or liquid water. For sealants, adhesives, and coatings, ordinary ambient humidity is usually adequate to promote cure. Heat or high humidity will accelerate cure, and low temperatures (e.g., 5°C or less) or low humidity (e.g., 15% relative humidity or less) will retard cure. Bonds to damp substrates (e.g., wood) typically cure faster than bonds to dry substrates (e.g., glass). The reactivity of a curable composition once it is exposed to water as a curing agent can be controlled by the amount of the compound of Formula I or Formula II present in the curable composition. A catalytically effective amount of the compound of Formula I or Formula II is the amount necessary to achieve the desired reactivity.

One of the most advantageous uses of the curable compositions of this invention is in orthopedic casting applications, where the composition is used as the resin component of a resin-coated flexible sheet, which resin component hardens on exposure to water. (As used herein, the term "coating" is intended to designate not only a surface application of composition, but also an application wherein a sheet material is impregnated with a composition, i.e., wherein the composition surrounds the fibers of the sheet material, or wherein the composition is absorbed by the fibers).

For use in orthopedic casting, the reactivity of the curable composition must not be so great that: (1) a hard film quickly forms on the surface of the composition preventing further penetration of the water into the bulk of the composition; or (2) the cast becomes rigid before the application and shaping is complete. The particular preferred amount of compound of Formula I or Formula II will depend upon the nature of the isocyanate-functional material, the desired set time, and the curing conditions. When the material is an isocyanate-functional polyurethane prepolymer based on an aromatic isocyanate, the amount of compound of Formula I or Formula II suitable for orthopedic casting applications will generally range from about 0.1% to about 5% by weight of the isocyanate-functional prepolymer, preferably from about 0.1 to about 3%, most preferably from about 0.2 to about 2%.

Foaming of the composition is preferably minimized because foaming reduces the porosity of the cast and its overall strength. Foaming occurs because carbon dioxide is released when water reacts with isocyanate groups. One way to minimize foaming is to add a foam suppressor such as ANTIFOAM™ A silicone fluid (Dow Corning), ANTIFOAM™ 1400 silicone fluid (Dow Corning), or L550 or L5303 silicone

surfactants. It is preferred to use a silicone liquid such as Dow Corning ANTIFOAM™ 1400 silicone fluid at a concentration of about 0.1 to 1.0 percent by weight.

The casting articles of this invention, useful as orthopedic casting tapes, comprise a flexible sheet material with a water-curable composition coated thereon. They are preferably prepared by forming an isocyanate-functional prepolymer in the presence of a compound of Formula I as described above and coating the resulting curable composition onto a flexible sheet material, e.g., a fabric.

In the preferred embodiments relating to casting articles, a porous, flexible sheet material is used. The porous material is preferably impregnated with the composition. A preferred example of a porous, flexible sheet material that can be impregnated with the composition of this invention is disclosed in U.S. Pat. No. 4,502,479. The sheet material disclosed therein imparts high structural strength to an orthopedic bandage prepared therefrom. A particularly preferred sheet material for use in the casting articles of this invention is the scrim used as the scrim component of SCOTCHCAST™ 2 Casting Tape (3M), described in U.S. Pat. No. 4,609,578, Example 1. The sheet material is a fiberglass fabric comprised of extensible knit fiberglass that exhibits an extensibility of at least about 20% in the length direction and has been heat set without tension in order to reduce fraying.

The amount of composition applied to the sheet material for use as a casting article such as an orthopedic casting tape must be sufficient for formation of a strong interlayer laminate bond but not so great as to occlude the porosity and unnecessarily thicken the sheet, which should be thin for rapid and complete hardening. Excessive composition can also cause the casting article to be messy to handle because of stickiness or dripping and transfer of composition.

The sheet material used in a casting article (e.g., an orthopedic casting tape) is generally formed in rolls of various widths, generally from 2.5 cm (one inch) to about 15 cm (six inches) wide. The sheet material can be coated with the curable composition in an amount, in terms of weight, of about 50 to about 500 $g/m^2$. In a preferred embodiment using a fiberglass fabric the curable composition preferably constitutes about 35% to about 50% by weight of the coated casting article. Generally, the composition will flow into the capillary spaces between contiguous filaments of the sheet material and will become rigidly bonded upon curing.

A casting article (e.g., an orthopedic casting tape) can be in the form of a roll wound up on a plastic core or in the form of a rolled or folded multi-layer laminate splint. The article can be sealed within a moisture- and oxygen-impermeable container such as an aluminum foil pouch. For use, the container is opened and the article is fully immersed and squeezed in tap water for about 5 to 30 seconds to replace entrapped air with water. Generally a sufficient amount of water is absorbed by the article in this manner. When a roll is unwound during wrapping of a cast, the excess moisture coats the freshly exposed composition surfaces insuring thorough wetting and rapid hardening. An alternate but less preferable method involves wrapping the cast without dipping and then allowing atmospheric moisture or water provided by spraying or by application of a wet towel to cure the composition.

Prior to applying an orthopedic cast to a limb or body member of a patient, a protective layer can be positioned about the limb or body member. The protective layer can take the form of a tubular stockinet or some other convenient form such as, for example, an elongate, non-woven, cotton, or polyester strip or bandage that can be wrapped about the limb or body member.

With the protective layer in a proper position, the moistened casting article can be wrapped about the body member and over the protective layer in a manner similar to that used in applying an elastic-type bandage. The cast can be shaped in a manner similar to that used in shaping a plaster-of-paris cast.

Eight or fewer layers of the cast material are generally sufficient to form a cast having significant strength within 8 minutes and having weight-bearing strength within 30 minutes. A fully cured cylindrical laminate having eight or fewer layers, e.g., six layers, should support at least about 3.6 kg/cm (20 lb/inch) and preferably at least about 7.2 kg/cm (40 lb/inch) of cylinder length according to the dry strength ring strength test described in detail below. A further test (delamination test) to illustrate strength is also described below.

<u>Ring Strength Test</u>

In this test, the "dry strength" of cured cylinders of resin-coated materials is determined. For this test, cured cylinders are formed as described below so as to form six-layered cylinders around a 2 inch (5.08cm) mandrel.

Each cylinder ring is formed by removing a roll of 3 inch (7.62cm) wide resin-coated material from its storage pouch and immersing the roll completely in deionized water having a temperature of about 80°F (27°C) for about 30 seconds. The roll of resin-coated material is then removed from the water and the material is wrapped around a 2 inch (5.08cm) mandrel covered with a thin stockinet to form six complete

uniform layers using a controlled wrapping tension of about 45 grams per centimeter width of the material. Each cylinder is completely wound within 30 seconds after removal of the roll from the water.

Thirty minutes after initial immersion in water, each cylinder is removed from its respective mandrel and allowed to cure for 48-60 hours in a controlled atmosphere of 75°F ± 3°F (34° ± 2°C) and 55% ± 5% relative humidity. Each cylinder is then placed in the fixture of an INSTRON™ tensile testing machine. Compression loads are applied to the cylinder along its exterior and parallel to its axis. The cylinder is placed lengthwise between the two bottom bars of the fixture (the bars being 1.9 centimeters wide, 1.3 centimeters in height, and 15.2 centimeters long, and spaced about 4 centimeters apart). The inside edges of the bars have a curbed surface having a 1/8 inch (0.31cm) radius. A third bar (0.63cm wide, 2.5cm high, and 15.2cm long) is then centered over the top of the cylinder, parallel to its axis. The contacting (bottom) edge of the third bar has a curved surface having a 1/8 inch (0.31cm) radius. The third bar is brought down to bear against and crush the cylinder at a speed of about 5 cm/min. The maximum or peak force applied while crushing the cylinder is recorded as the ring strength, which in this particular instance is the "dry strength" (expressed in terms of force per unit length of the cylinder, i.e., newtons/cm). For each material, at least five samples are tested, and the average peak force applied is calculated and reported as the "dry strength".

To measure the "wet strength", the same procedure is followed as for the "dry strength", except that after curing for 48-60 hours, the cylinder is immersed in water at about 113°F (45°C) for about 30 minutes, and then allowed to dry under ambient conditions for about 15 minutes. The cylinder is then placed in the instrument and crushed as described above in order to determine "wet strength."

To determine the "warm wet strength" of the cylinder, the procedure as set forth above for "wet strength" is followed, except that the cylinder is placed in the fixture and crushed immediately after removal from the 113°F (45°C) water bath.

Delamination Test

This test measures the force necessary to delaminate a cured cylinder of a resin-coated material.

Each cylinder ring is formed by removing a roll of 3 inch (7.62 cm) wide resin-coated material from its storage pouch and immersing the roll completely in deionized water having a temperature of about 80°F (27°C) for about 30 seconds. The roll of resin-coated material is then removed from the water and the material is wrapped around a 2 inch diameter (5.08 cm) mandrel covered with a thin stockinet to form six complete uniform layers using a controlled wrapping tension of about 45 grams per centimeter width of the material. A free tail about 6 inches (15.24 cm) long is kept and the rest of the roll is cut off. Each cylinder is completely wound within 30 seconds after removal of the roll from the water.

Fifteen to 20 minutes after initial immersion of the roll in water, the cured cylinder is removed from the mandrel. Delamination strength is determined as follows:

The free tail of the cylinder is placed in the jaws of an INSTRON™ Model 1122 tensile testing machine and a spindle is placed through the hollow core of the cylinder so that the cylinder rotates freely about the axis of the spindle. The tensile testing machine is then activated to pull on the free tail at a speed of 127 cm/min. The average force required to delaminate the wrapped layers over the first 33 centimeters of the material is recorded in terms of force per unit width of sample (newtons/cm). For each material, at least five samples are tested, and the average delamination force is calculated and reported as the "delamination strength."

In addition to the above-described use in orthopedic casting, curable compositions of this invention will be useful in a variety of applications wherein isocyanate-functional materials have been used previously, e.g., as sealants (e.g., caulks), coatings, foams, adhesives, and so forth. They can be applied to a variety of articles and substrates, such as articles or substrates of glass, metal, plastic, wood, leather, masonry, textiles, and the like.

When used as an adhesive, the composition is placed between an article and a substrate, in contact with both, and exposed to moisture sufficient to cure the composition. When used as a coating, the composition is deposited as a continuous layer on the surface of the article to be coated and exposed to moisture sufficient to cure the composition. When used as a sealant, the composition is deposited in the void to be sealed and exposed to moisture sufficient to cure the composition. When used as a structural reinforcing material, the composition is coated onto and/or impregnated into an article comprised of a flexible sheet of fibrous or non-fibrous fabrics, papers, felts, foams and the like and exposed to moisture sufficient to cure the composition. When used for making foams the compositions are usually mixed with a precise amount of water and immediately packed into an appropriate mold. For such applications wherein the isocyanate-functional material is an isocyanate-functional polyurethane prepolymer based on an aromatic isocyanate, an effective amount of compound of Formula I preferably is about 0.002 to 2 weight percent, and most preferably about 0.05 to 0.5 weight percent based upon the weight of prepolymer.

Other ingredients and adjuvants can be incorporated into the compositions of the invention. Suitable ingredients and adjuvants and effective amounts thereof are disclosed, e.g. in U.S. Pat. No. 4,705,840 (Buckanin) and are easily selected by those skilled in the art.

The compositions of the invention can be put into packages according to techniques known to those skilled in the art. Suitable packages include, for example, aluminum foil laminate pouches, caulking tubes (made, for example, of aluminum foil laminates, metal or plastic), screw-capped squeezable tubes, cans, drums, and the like.

Several compounds of Formula I were prepared as follows:

Catalyst A. 2-(N,N-Dimethylamino)ethyl N,N-Dimethylaminoacetate

A mixture of 65.5 g (0.5 mol) of ethyl N,N-dimethylglycinate, 44.6 g (0.5 mol) of N,N-dimethylethanolamine and 0.82 g of dibutyltin oxide was heated at about 130 °C. The ethanol by-product was collected in a Dean-Stark trap. When the rate of ethanol evolution and amount of ethanol collected showed that the reaction was essentially complete, the reaction was heated at about 180 °C until ethanol evolution ceased. The liquid product was purified by distillation at reduced pressure to provide 2-(N,N-dimethylamino)ethyl N,N-dimethylaminoacetate.

Catalyst B. 2-(1-Piperidino)ethyl N,N-Dimethylaminoacetate

Using the method described for Catalyst A, 65.6 g ethyl N,N-dimethylglycinate was reacted with 64.6 g of 2-(1-piperidino)ethanol and 0.97 g of dibutyltin oxide to provide the desired product, which was purified by distillation.

Catalyst C. 2-(N,N-Dimethylamino)ethyl N-methylpipecolinate

A mixture of 85 g (0.5 mol) of ethyl 1-methylpipecolinate, 44.6 g (0.5 mol) of N,N-dimethylethanolamine and 0.82 g of dibutyltin oxide was heated at about 145 °C for 30 hours. The ethanol by-product was collected in a Dean-Stark trap. When the rate of ethanol evolution and amount of ethanol collected showed that the reaction was essentially complete, the reaction was heated at about 180 °C until ethanol evolution ceased. The liquid product was purified by distillation at reduced pressure to provide 2-(N,N-dimethylamino)ethyl N-methylpipecolinate.

Catalyst D. N-Methyl-(2-piperidyl)methyl N-Methylpipecolinate

A mixture of 85.6 g (0.5 mole) of ethyl 1-methylpipecolinate, 64.6 g (0.5 mole) of 1-methyl-2-piperidinemethanol and 1.1 g dibutyltin oxide was heated at 130 °C for three hours and then gradually heated to 200 °C. When the amount of ethanol collected indicated the reaction was essentially complete the liquid product, N-methyl-(2-piperidyl)methyl N-methylpipecolinate, was distilled at 0.25 mm Hg pressure at about 130 °C. The nuclear magnetic resonance and infrared spectra of the product were consistent with the structural assignment.

Catalyst E. N-Methyl-(2-piperidyl)methyl N,N-Dimethylaminoacetate

A mixture of 75 g of ethyl N,N-dimethylglycinate, 75 g of 1-methyl-2-piperidylmethanol and 1 g of titanium tetra(n-butoxide) was heated at 145 °C until the amount of ethanol collected indicated the reaction was complete. After cooling a small amount of water was added, the mixture was filtered, and the filtrate was distilled under reduced pressure to provide N-methyl-2-piperidylmethyl N,N-dimethylaminoacetate.

Catalyst F. 2-(N-Morpholino)ethyl 2-(N-Morpholino)propionate

To 174 g of morpholine was added slowly with stirring 181 g of ethyl 2-bromopropionate. To the stirred mixture was added 200 ml of diethyl ether while maintaining the temperature about 35 °C. After about 16 hours 700 ml of toluene was added, then the insoluble salts were removed by filtration. The salts were neutralized by the addition of potassium carbonate, then extracted with toluene. The toluene filtrate and extracts were evaporated to provide 158 g of ethyl 2-(N-morpholino)propionate.

To 50 g of ethyl 2-(N-morpholino)propionate was added 50 g of N-(2-hydroxyethyl)morpholine and 0.5 g of dibutyltin oxide. This mixture was heated to 150 ° C. A Dean Stark trap was used to collect evolved ethanol. After 2 hours the temperature was elevated to 175 ° C and maintained at that temperature for 3 hours. After a total of 14 ml of ethanol had been collected, the product was purified by vacuum distillation to provide 2-(N-morpholino)ethyl 2-(N-morpholino)propionate as a crystalline solid.

Catalyst G. 2-(N-Morpholino)ethyl N,N-Diethylaminoacetate

To a mixture of 70 g of N-[(2-hydroxy)ethyl]morpholine and 85 g of ethyl (N,N-diethylamino)acetate was added 0.8 g of dibutyltin oxide and the mixture was heated to 145 ° C. A Dean Stark trap was used to collect the evolved ethanol. An additional 0.4 g of dibutyltin oxide was added and the temperature was increased gradually to 180 ° C over several hours. The total volume of ethanol collected was 28 ml. The product, 2-(N-morpholino)ethyl N,N-diethylaminoacetate, was separated by vacuum distillation. The structural assignment was confirmed by nuclear magnetic resonance and infrared spectral analysis.

Catalyst H. 2-(N-Morpholino)ethyl N,N-Dimethylaminoacetate

Using the general method used to prepare Catalyst G but using ethyl (N,N-dimethylamino)acetate, the product 2-(N-morpholino)ethyl N,N-dimethylaminoacetate was obtained by vacuum distillation.

Catalyst I. 2-(1-Pyrrolidino)ethyl 1-Methylpipecolinate

Using the general method used to prepare Catalyst G but using N-(2-hydroxyethyl)pyrrolidine and ethyl 1-methylpipecolinate the product 2-(1-pyrrolidino)ethyl 1-methylpipecolinate was obtained by vacuum distillation.

Catalyst J. 2-(1-Pyrrolidino)ethyl N,N-Dimethylaminoacetate

Using the general method used to prepare Catalyst G but using N-(2-hydroxyethyl)pyrrolidine and ethyl N,N-dimethylaminoacetate the product 2-(1-pyrrolidino)ethyl N,N-dimethylaminoacetate was obtained by vacuum distillation.

pKa

Several catalysts were characterized by their titration curves (i.e., their pKa values) as follows:

To a 0.1 g sample of a catalyst was added with stirring 60 ml of distilled water, then 10 ml of 0.1N hydrochloric acid was added. The solution was stirred for 10 minutes, then backtitrated with 0.1N sodium hydroxide solution. The pH during backtitration was measured potentiometrically using a Metrohm Model 670 TITROPROCESSOR™ potentiometer (Brinkman Instruments Inc., Westbury, NY). The endpoint and half-neutralization point were determined by the instrument, and for the purposes of this determination the half-neutralization point is taken as the pKa. The values of pKa for several catalysts are shown in Table I below.

**TABLE I**

| Catalyst | Measured pKa First | Measured pKa Second |
|---|---|---|
| 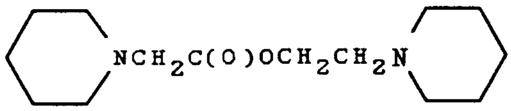 | 8.97 | 6.72 |
| $(CH_3)_2NCH_2C(O)OCH_2CH_2N(CH_3)_2$ | 8.53 | 6.49 |
| 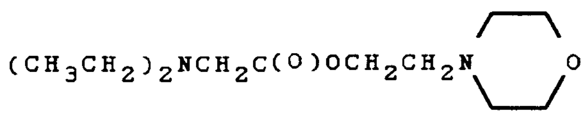 | 7.56 | 5.36 |
| 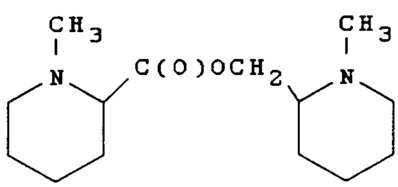 | 8.85 | 6.41 |
| 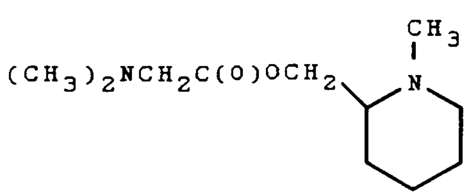 | 9.05 | 6.4 |

The following Examples are provided to illustrate the invention and are not intended to limit the scope of the invention. Parts and percentages are by weight unless otherwise indicated, and temperatures are designated in degrees Celsius.

Examples 1-9

Compositions of the invention useful, for example, in connection with orthopedic casting materials, sealants, adhesives, foams, or coatings, were prepared and their gel times were measured as set forth below:

To a stirred 30.00 g sample of ISONATE™ 2143L isocyanate (Dow) in a 100 mL (4.5 cm diameter) polyethylene beaker was added 0.30 g of a selected catalyst, then 10 ml of water was added gently from a syringe. The mixture was stirred by hand with a 1.7 cm wide wooden tongue depressor at a rate of about

60 to 75 rpm. This time until gelation occurred was recorded. Gelation was defined as the point at which the viscosity was sufficient to allow formation of a permanent depression in the mixture.

Results are shown in Table II below.

**TABLE II**

| Catalyst | Example | Gel Time (Sec.) |
|---|---|---|
| 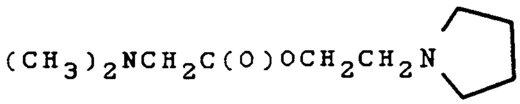 | 1 | 28 |
| 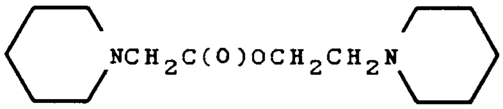 | 2 | 85 |
| $(CH_3)_2NCH_2C(O)OCH_2CH_2N(CH_3)_2$ | 3 | 71 |
| 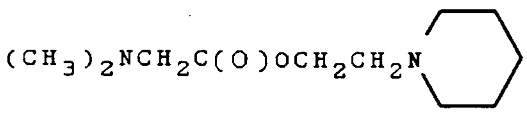 | 4 | 120 |
| 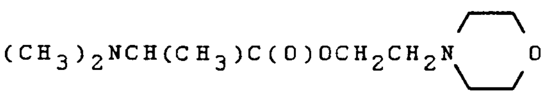 | 5 | 184 |
| 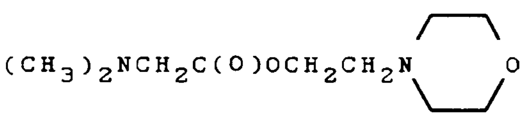 | 6 | 213 |
| 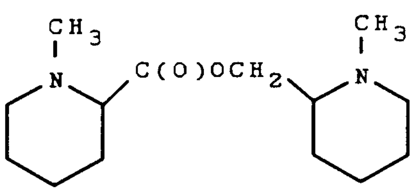 | 7 | 35 |

**TABLE II (Cont.)**

| Catalyst | Example | Gel Time (Sec.) |
|---|---|---|
| 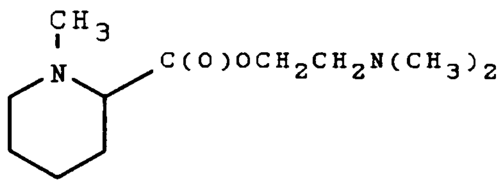 | 8 | 71 |
| 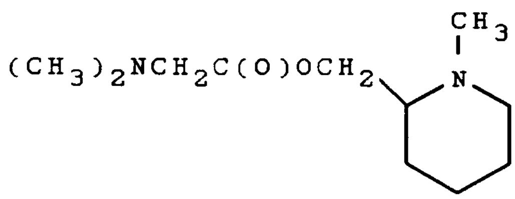 | 9 | 38 |

Example 10

A curable composition of the invention useful, for example, in connection with an orthopedic casting material, a sealant, an adhesive, a foam, or a coating, was prepared as follows:

An amount, 1125 g (7.76 equivalents), of ISONATE™ 2143L isocyanate (modified diphenylmethane diisocyanate, Dow) was added to a one gallon (4L) jar having a three-necked lid equipped with a thermometer, stirrer and nitrogen inlet. To this was added 30.0 g (about 1.5 weight percent) of 2-(1-piperidino)ethyl N,N-dimethylaminoacetate (Catalyst B), 1.0 g of paratoluenesulfonyl chloride, 80 g of PLURONIC™ F-108 polyethylene oxide-polypropylene oxide block copolymer (available from BASF Wyandotte Corporation), and 3.6 g of Dow-Corning DB-100 silicone fluid. This was followed by the addition of 9.6 g of BHT (2,6-di-tert-butyl-4-methylphenol) in 480 g of NIAX™ PPG-725 (a polypropylene oxide polyol having a molecular weight of about 750, AC West Virginia Polyol Co.) and 270 g of NIAX™ PPG-425 (a polypropylene oxide polyol having a molecular weight of about 425, AC West Virginia Polyol Co.). The theoretical equivalent ratio of isocyanato groups to hydroxyl groups was 3.15:1 and the isocyanato equivalent weight of the resulting prepolymer was 375 g/equivalent. The addition of the polyol mixture was made through a dropping funnel over a period of thirty minutes. After addition the polymerization reaction was carried out at 50-60 °C for one hour to afford a curable composition of the invention.

Example 11

Using the general method of Example 10, 2-(N,N-dimethylamino)ethyl N,N-dimethylaminoacetate (Catalyst A) was incorporated (in an amount of about 1.0 percent by weight) into a curable composition of the invention.

Examples 12-15 and Comparative Examples C-1 and C-2

As shown in Table III below, curable compositions of the invention useful, for example, in connection with orthopedic casting materials, sealants, adhesives, foams, or coatings, and comparative compositions were prepared in the general manner described in Example 10. While still hot (49-60 °C) the compositions were poured into 240 mL (8 oz) jars and the jars were sealed. The samples were subjected to accelerated aging conditions of 49 °C in the sealed glass jars. Individual samples were periodically cooled to room

temperature and then equilibriated for at least 4 hours in a water bath at 23° C. The viscosity of the cooled sample was measured using a SYNCHROLECTRIC™ Viscometer Model RVT (Brookfield Engineering Labs, Inc., Spoughton, Massachusetts) using spindles #6 and #7. Viscosity values are set forth in Table III below.

## TABLE III

| | Example | | | | | |
|---|---|---|---|---|---|---|
| | 12 | 13 | 14 | 15 | C-1 | C-2 |
| | Catalyst (concentration in weight percent) | | | | | |
| | D | H | A | *4 | ** | *** |
| Day | (0.5%) | (3.8%) | (1.0%) | (1.75%) | (1.32%) | (0.5%) |
| | Viscosity (cps x $10^{-3}$) | | | | | |
| 0 | 77 | 64 | 70.5 | 63.5 | 65 | 67 |
| 4 | 57 | 52 | 53.5 | 55 | 50 | 138 |
| 10 | 64.5 | 60 | 69.5 | 69.5 | 62 | 830 |
| 15 | 83 | 67 | 93 | 87 | 69 | >2000 |
| 20 | 86 | 73 | 94 | 92.5 | 73 | NT |
| 28 | 102 | 86 | 124 | 112 | 82 | NT |
| 33 | 128 | 102 | 164 | 156 | 98 | NT |
| 38 | 140 | 126 | 184 | 196 | 112 | NT |
| 43 | 136 | NT | 202 | 194 | 96 | NT |
| 48 | 160 | NT | NT | 224 | 112 | NT |

NT = Not Taken

* 2-(N-piperidyl)ethyl N-piperidyl acetate

** morpholinoethyl morpholinoisopropyl ether

*** $[CH_3)_2NCH_2CH_2]_2O$

The results in Table III show that these compositions of the invention age well compared to Comparative Example C-2.

Examples 16-17 and Comparative Examples C-3 and C-4

Curable compositions of the invention useful, for example, in connection with orthopedic casting materials, sealants, adhesives, foams, or coatings, and comparative compositions were prepared as described in general terms in Example 10 using various catalysts as shown in Table IV below. The amount of catalyst used was selected to provide a set time of about 3 minutes.

240 mL (8 oz) samples of the compositions of Examples 16 and 17 and Comparative Examples C-3 and C-4 were subjected to accelerated aging conditions of 49°C in sealed glass jars. Individual samples were periodically cooled to room temperature and then equilibriated for 2 hours in a water bath at 23°C. The viscosity of the cooled sample was measured using a SYNCHRO-LECTRIC™ Viscometer Rodel RVT (Brookfield Engineering Labs, Inc., Spoughton, Massachusetts) using spindles #6 and #7. The viscosities are set forth in Table IV below.

**TABLE IV**

| | Example | | | |
|---|---|---|---|---|
| | 16 | 17 | C-3 | C-4 |
| | Catalyst (concentration in weight percent) | | | |
| | E | B | * | ** |
| Day | (0.5%) | (1.6%) | (2.3%) | (1.32) |
| | Viscosity (cps x $10^{-3}$) | | | |
| 0 | 75 | 53 | 62.5 | 48 |
| 7 | 84 | 66 | 74 | 52 |
| 12 | 94 | 78 | 86 | 64 |
| 17 | 108 | 92 | 108 | 68 |
| 28 | 104 | 92 | 86 | 66 |
| 35 | 114 | 102 | 98 | 71.5 |
| 42 | 130 | 126 | 108 | 74 |
| 49 | 155 | 166 | 164 | 96 |

* 2,2'-Dimorpholinodiethyl ether

** Morpholinoethyl morpholinoisopropyl ether

The results in Table IV show that upon accelerated aging these casting articles of the invention exhibit viscosity increases comparable to those of the Comparative Examples.

The following Examples are provided to illustrate the synthesis of novel compounds of Formula II of the invention.

Example 18 Ethyl 1-Pyrrolidineacetate

To an ice bath-cooled solution of 213.4 g (3 mol) of pyrrolidine in 450 mL of tetrahydrofuran was added dropwise over about 2 hours 250.5 g (1.5 mol) of ethyl bromoacetate, maintaining the temperature below 30 °C. To this solution was added 150 mL of toluene to precipitate pyrrolidinium bromide, which was separated by filtration. The filtrate was evaporated under vacuum to provide a residue of about 241 g of ethyl 1-pyrrolinidineacetate.

Example 19 2-(1-Piperidino)ethyl1-Pyrrolidineacetate

A mixture of 78.5 g (0.5 mol) of ethyl 1-pyrrolidineacetate, 64.6 g (0.5 mol) of 2-piperidinoethanol and 1.0 g of dibutyltin oxide was heated at about 150 °C while collecting the ethanol by-product in a Dean-Stark trap. When the rate of ethanol evolution and amount of ethanol collected showed that the reaction was essentially complete, the reaction was heated at about 170 °C until ethanol evolution ceased. The liquid product was purified by distillation at reduced pressure to provide 2-(1-piperidino)ethyl 1-pyrrolidineacetate.

Example 20 2-(4-Morpholino)ethyl 1-Pyrrolidineacetate

Using the method of Example 2, 75.5 g ethyl 1-pyrrolidineacetate was reacted with 65.6 g of 4-(2-hydroxyethyl)morpholine to provide the desired product, which was purified by distillation at a temperature of about 120 °C and a pressure of about 300 μmHg.

Example 21 2-(N,N-Dimethylamino)ethyl 1-Pyrrolidineacetate

To a mixture of 80 g (0.51 mol) of ethyl 1-pyrrolidineacetate and 0.5 g of sodium hydride (in the form of a 60% by weight suspension in mineral oil) was added 48 g (0.54 mol) of 2-(N,N-dimethylamino)ethanol. The mixture was purged with nitrogen gas, heated gradually and then maintained at about 150 °C while collecting the ethanol by-product in a Dean-Stark trap. The liquid reaction product was distilled at 85 °C at a pressure of 100 µmHg to provide 2-(N,N-dimethylamino)-ethyl 1-pyrrolidineacetate. The structure of the product was confirmed by nuclear magnetic resonance and infrared spectral analysis.

Example 22 2-(1-Pyrrolidinyl)ethyl 1-Pyrrolidineacetate

To a mixture of 1.3 g of sodium hydride (in the form of a 60% by weight suspension in mineral oil) and 100 g (0.64 mol) of ethyl 1-pyrrolidineacetate was added 91 g of 2-(1-pyrrolidinyl)ethanol. The mixture was heated slowly to about 225 °C, and the ethanol by-product was collected in a Dean-Stark trap. The reaction mixture was cooled to about 25 °C, then 12 g of 1-bromopropane was added to react any alkoxide present. The sodium bromide by-product was removed by filtration. The desired product 2-(1-pyrrolidinyl)ethyl 1-pyrrolidineacetate was purified by distillation at 95 °C at a pressure of 250 µmHg. The structural assignment was confirmed by mass spectral analysis.

Example 23 2-(N,N-Diethylamino)ethyl 1-Pyrrolidineacetate

Using the method of Example 22, ethyl 1-pyrrolidineacetate was reacted with 2-(N,N-diethylamino)-ethanol to provide 2-(N,N-diethylamino)ethyl 1-pyrrolidineacetate, b.p. 92 °C at 250 µmHg.

Example 24 2-(1-Methylpiperidyl)methyl 1-Pyrrolidineacetate

Using the method of Example 22, ethyl 1-pyrrolidineacetate was reacted with 2-(1-methylpiperidyl)-methanol to provide 2-(1-methylpiperidyl)methyl 1-pyrrolidineacetate. Purification was carried out by distillation at 125 °C and at a pressure of 400 µmHg.

Example 25 N-(N-Acetylpiperazinyl)ethyl 1-Pyrrolidineacetate

Using the method of Example 21, 43.2 g of ethyl 1-pyrrolidineacetate was reacted with 42.5 g of N-(2-hydroxyethyl)-N'-acetylpiperazine in the presence of 0.7 g of 60 weight percent sodium hydride in mineral oil. The product was purified by distillation at a temperature of about 180 °C and a pressure of about 0.5 mmHg. Gas chromatography of the product showed a single major constituent.

Examples 26-28

Curable compositions useful, for example, in connection with orthopedic casting materials, sealants, adhesives, foams, or coatings, comprising a compound of the invention of Formula II were prepared and their gel times measured as set forth below:

To a stirred 30.00 g sample of ISONATE™ 2143L isocyanate (Dow) in a 100 mL (4.5 cm diameter) polyethylene beaker was added 0.30 g of a compound of Formula II, then 10 mL of water was added gently from a syringe. The mixture was stirred by hand with a 1.7 cm wide wooden tongue depressor at a rate of about 60 to 75 rpm. The time until gelation occurred was recorded as gel time. Gelation was defined as the point at which the viscosity was sufficient to allow formation of a permanent depression in the mixture.

The gel time was found to be 45 seconds for the composition of Example 26, comprising 2-(1-piperidino)ethyl 1-pyrrolidineacetate, 90 seconds for the composition of Example 27, comprising 2-(4-morpholino)ethyl 1-pyrrolidineacetate, and 400 seconds for the composition of Example 28, comprising for N-(N-acetylpiperazinyl)ethyl 1-pyrrolidineacetate.

Example 29

A further curable composition useful, for example, in connection with an orthopedic casting material, a sealant, an adhesive, a foam, or a coating, was prepared as follows:

An amount, 1125 g (7.76 equivalents), of ISONATE™ 2143L isocyanate (modified diphenylmethane diisocyanate, Dow) was added to a one gallon (4L) jar having a three-necked lid equipped with a

thermometer, stirrer and nitrogen inlet. To this was added 29.99 g of the catalyst 2-(1-piperidino)ethyl 1-pyrrolidineacetate from Example 19, 1.0 g of para-toluenesulfonyl chloride, 80 g of PLURONIC™ F-108 polyethylene oxide-polypropylene oxide block copolymer (available from BASF Wyandotte Corporation), and 3.6 g of Dow-Corning DB-100 silicone fluid. This was followed by the addition of 9.6 g of BHT (2,6-di-tert-butyl-4-methylphenol) in 480 g of NIAX™ PPG-725 (a polypropylene oxide polyol having a molecular weight of about 750, AC West Virginia Polyol Co.) and 270 g of NIAX™ PPG-425 (a polypropylene oxide polyol having a molecular weight of about 425, AC West Virginia Polyol Co.). The addition of the polyol mixture was made through a dropping funnel over a period of thirty minutes. After addition the polymerization reaction was carried out at 50-60 °C for one hour to afford a curable composition of the invention.

Examples 30-32 and Comparative Examples C-5 through C-7

In the manner of Example 29 above, compounds of Formula II and known catalysts were independently incorporated into curable compositions as set forth in Table V below, wherein catalyst concentration is given in weight percent based on the total weight of the curable composition.

Table V

| Example | Catalyst | Example Number | Concentration (%) |
|---|---|---|---|
| 30 | 2-(N,N-diethylamino)ethyl 1-pyrrolidineacetate | 23 | 0.9 |
| 31 | 2-(N,N-dimethylamino)ethyl 1-pyrrolidineacetate | 21 | 0.8 |
| 32 | 2-(1-pyrrolidinyl)ethyl 1-pyrrolidineacetate | 22 | 0.75 |
| C-5 | DMDEE™ (Texaco)[A] | -- | 2.5 |
| C-6 | MEMPE[B] | -- | 1.32 |
| C-7 | NIAX™ A-99[C]/DMEA[D] | -- | 0.3/0.3 |

A. 2,2'-Dimorpholinodiethyl ether
B. Morpholinoethyl morpholinoisopropyl ether
C. $[(CH_3)_2NCH_2CH_2]_2O$, AC West Virginia Polyol Co.
D. Dimethylethanolamine

Examples 33-34

Further curable compositions useful, for example, in connection with orthopedic casting materials, sealants, adhesives, foams, or coatings, were prepared according to the general method of Example 29 above. The particular catalyst used and the amount thereof in weight percent based on total weight of the composition are shown in Table VI. The amount of catalyst used was selected to provide a set time of approximately 3 minutes. About 2.5 to 3 kg of each composition was made, mixed for about 2 hours, and sealed in several 240 mL (8 oz) glass jars.

Table VI

| Example | Catalyst | Example Number | Concentration (%) |
|---|---|---|---|
| 33 | 2-(1-methylpiperidino)methyl 1-pyrrolidineacetate | 24 | 0.6 |
| 34 | 2-(N,N-dimethylamino)ethyl 1-pyrrolidineacetate | 21 | 0.9 |

Aging Data

The compositions of each of Examples 30-32 and Comparative Examples C-5 through C-7 were independently machine coated using a curtain coating technique in an atmosphere substantially free of moisture (less than 5% relative humidity) on a 7.6 cm (three inch) wide strip of heat-set fiberglass fabric [the fabric of SCOTCHCAST PLUS™ casting tape, 3M, described in U.S. Pat. No. 4,609,578 (Reed)] to give a casting article in the form of a tape containing 42.5% by weight of the composition based on total weight of the tape. The tape was then cut in lengths of about 3.65 m (4 yards), rolled onto 7.6 cm (3 inch) long

hollow cylindrical polyethylene cores having a diameter of 1.9 cm (0.75 inch), and packaged in foil pouches for storage until later use and evaluation. Rolled samples of the casting article prepared above were stored in sealed aluminum foil envelopes similar to those used to store similar commercial items. The casting articles were subjected to accelerated aging at a temperature of 65.5°C (150°F) for the period of time indicated in Table VII below. After cooling to 23-25°C and equilibrating for 24 hours, the rolls were sequentially removed from the pouches and immediately unwound in such a manner that the force needed to unwind the rolls could be measured.

The force needed to unwind the rolls was measured using an Instron™ Model 1122 tensile testing machine with a 50lb (22.7Kg) load cell. Each roll was unwound counterclockwise from a freely rotating spindle over a freely rotating crosshead spindle attached to the load cell of the tensile testing machine onto a 3.875 inch (9.84cm) diameter take-up roller rotating at 60 revolutions per minute covered with stockinette (3M MS04). The crosshead spindle consisted of a freely rotating 3/8 inch dia x 5.5 inch (0.95cm x 14cm) long rod counter-balanced to ensure that it hung in a horizontal position parallel to both the unwind spindle and the take-up roller. The force was measured by a Microcon™ Model MC4100 microprocessor using the following machine conditions:

Area = 0
gage length = 4.5 inches (11.4cm)
crosshead speed = 0.1 inches/min (0.254cm/min)
Start force averaging at 0.0005 inches (0.00127cm)
(preset point 8, elongation, 0.005)
End force averaging at 0.0136 inches (0.0345cm) (preset point 9, elongation, 0.0136)
Fail Criteria = 100%
Load Limit = 43,360 kg force
Crosshead stop = off
Elongation correction factor = no correction

The average force over 95.7 inches (24.3cm) of tape was recorded as the unwind force (ignoring the first 3.65 inches (9.3cm) of tape).

The unwind force was measured for 5 rolls and the average values are set forth in TABLE VII below, wherein a lack of an entry indicates that the roll was cured when tested.

Table VII

| Unwind Force (Newtons) | | | | | | |
|---|---|---|---|---|---|---|
| Day | Example | | | | | |
| | 30 | 31 | 32 | C-5 | C-6 | C-7 |
| 0 | 6.85 | 6.05 | 6.41 | 5.6 | 8.76 | 8.81 |
| 7 | 11.0 | 9.46 | 10.1 | 8.65 | 11.0 | -- |
| 16 | 20.3 | 37.7 | 18.6 | 13.6 | 15.8 | -- |
| 24 | 48.1 | -- | 38.6 | 19.8 | 27.5 | -- |

The results in Table VII show that upon accelerated aging these casting articles comprising a catalyst of this invention exhibit unwind tension comparable to, and often superior to, those of the comparative examples.

240 mL (8 oz) samples of the compositions of Examples 32-34 and Comparative Examples C-5 and C-6 above were subjected to accelerated aging conditions of 49°C in sealed glass jars. Individual samples were periodically cooled to room temperature and then equilibriated for 2 hours in a water bath at 23°C. The viscosity of the cooled sample was measured using a SYNCHRO-LECTRIC™ Viscometer Model RVT (Brookfield Engineering Labs, Inc., Spoughton, Massachusetts) using spindles #6 and #7. The viscosities are set forth in Table VIII below.

Table VII

| Viscosity (cps x $10^{-3}$) | | | | | |
|---|---|---|---|---|---|
| Day | Example | | | | |
| | 32 | 33 | 34 | C-5 | C-6 |
| 0 | 75 | 72 | 69 | 66 | 58 |
| 7 | 80 | 74 | 81 | 86 | 58 |
| 14 | 120 | 96 | 98 | 88 | 72 |
| 21 | 138 | 96 | 106 | 90 | 72 |
| 28 | 204 | 124 | 130 | 108 | 78 |
| 35 | 300 | 152 | 170 | 120 | 82 |
| 42 | 470 | 150 | 232 | 116 | 80 |
| 49 | 590 | 208 | 266 | 128 | 72 |

pKa

Several of the compounds of the invention were characterized by their titration curves (i.e., their pKa values) as follows:

To a 0.1 g sample of a compound of the invention was added with stirring 60 mL of distilled water, then 10 mL of 0.1N hydrochloric acid was added. The solution was stirred for 10 minutes. The pH of the solution was then measured potentiometrically using a Metrohm Model 670 TITROPROCESSOR™ potentiometer (Brinkman Instruments Inc., Westbury, NY) while backtitrating with 0.01N sodium hydroxide solution. The endpoints and half-neutralization points were determined by the instrument, and for the purposes of this determination the half-neutralization point is taken as the pKa. The values of pKa for several compounds are as shown in Table IX.

Table IX

| Compound | MeasupKa red | |
|---|---|---|
| | first | second |
| 2-(1-piperidino)ethyl 1-pyrrolidineacetate | 8.97 | 6.72 |
| 2-(1-pyrrolidino)ethyl 1-pyrrolidineacetate | 9.26 | 7.12 |
| 2-(1-methylpiperidinyl)methyl 1-pyrrolidineacetate | 9.15 | 7.03 |

Example 35

A curable composition useful, for example, in connection with an orthopedic casting material, a sealant, an adhesive, a foam, or a coating, was prepared as follows:

An amount, 2210 g (15.35 equivalents), of ISONATE™ 2143L isocyanate (modified diphenylmethane diisocyanate, Dow) was added to a one gallon (4L) jar having a three-necked lid equipped with a thermometer, heating mantle, stirrer, and nitrogen inlet. To this was added 1.8 g of benzoyl chloride, 6.66 g of Dow-Corning DB-100 silicone fluid, 17.76 g of BHT (2,6-di-tert-butyl-4-methylphenol), 148 g of PLURONIC™ F-108 polyethylene oxide-polypropylene oxide block copolymer (available from BASF Wyandotte Corporation) and 22.2 g of 2-(1-methylpiperidyl)methyl 1-pyrrolidineacetate (Example 7). This was followed by the addition of 620.1 g of NIAX™ Polyol PPG-2025 (a polypropylene oxide polyol having a molecular weight of about 2025, available from AC West Virginia Polyol Co.), 229.4 g of LG 650 polyol (a polyol containing polymeric particles, available from AC West Virginia Polyol Co.), and 444 g of NIAX™ E-562 (a polypropylene oxide available from AC West Virginia Polyol Co.). The polymerization reaction was carried out at 50-60 °C for one hour to afford a curable composition.

Example 36 and Comparative Example C-8

Using the general method of Example 35, 2-(1-pyrrolidino)ethyl 1-pyrrolidineacetate (Example 22) was incorporated into the curable composition of Example 36. As Comparative Example C-8, a composition using MEMPE as a catalyst was prepared. The ingredients and amounts used in Example 36 and Comparative Example C-8 are shown in Table X.

Table X

| Component | Example 36 | Comparative Example C-8 |
|---|---|---|
| | Weight (g) | Weight (g) |
| ISONATE™ 2143L | 2204.46 | 2183.9 |
| Benzoyl Chloride | 1.85 | 0 |
| p-Toluenesulfonyl Chloride | 0 | 1.8 |
| DB-100 | 6.66 | 66.6 |
| BHT | 17.76 | 17.75 |
| Catalyst | 27.75 | 48.81 |
| PLURONIC™ F-108 | 148.0 | 147.91 |
| NIAX™ PPG-2025 | 620.12 | 620.12 |
| LG 650 | 229.40 | 229.26 |
| NIAX™ E-562 | 444.00 | 443.74 |
| TOTAL | 3700.00 | 3700.00 |

The compositions of Examples 35 and 36 and Comparative Example C-8 were machine coated as described above in connection with "Aging Data". The resulting casting tapes were packaged in foil pouches for storage until later use and evaluation.

Using the tests described above, "dry strength", "wet strength", "ring delamination", and "warm wet strength" were measured for casting tapes comprising Compositions 35 and 36 and Comparative Composition C-8. The results are shown in Table XI below:

Table XI

| Strength Test | Example | | |
|---|---|---|---|
| | 35 | 36 | C-8 |
| Dry | 911.9 | 900.8 | 863.8 |
| Wet | 696.6 | 703.7 | 492 |
| Warm Wet | 528.4 | 524.9 | 285.1 |
| Ring Delamination | 56.5 | 53.4 | 51.2 |
| (Strength measured in newtons) | | | |

The results in Table XI show that, when used in curable compositions and casting tapes, compounds of this invention provide useful products that exhibit significantly higher wet strength, warm wet strength, and ring delamination values than the Comparative Example.

## Claims

1. A curable composition comprising (i) an isocyanate-functional material; and (ii) a catalytically effective amount of a compound: (A) of the formula

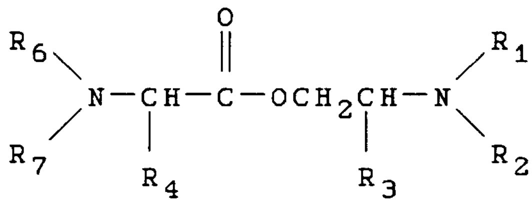

wherein

$R_1$ and $R_2$ are independently straight chain, branched chain, or cyclic alkyl of one to six carbon atoms, or $R_1$ and $R_2$ together form a straight chain or branched chain alkylene group having four or five carbons in the main alkylene chain, or $R_1$ and $R_2$ together form a group of the formula -A-O-B- or

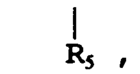 ,

wherein A and B are independently straight chain or branched chain alkylene groups each having two carbon atoms in their main alkylene chain and $R_5$ is alkyl, aryl, or a deactivating substituent;

$R_3$ is hydrogen or straight chain, branched chain, or cyclic alkyl of one to six carbon atoms, or $R_1$ and $R_3$ together form a straight chain or branched chain alkylene group having three or four carbon atoms in the main alkylene chain, with the proviso that when $R_1$ and $R_3$ together form a straight chain or branched chain alkylene group having three or four carbon atoms in the main alkylene chain, then $R_2$ is straight chain, branched chain, or cyclic alkyl of one to about six carbon atoms;

$R_4$ is hydrogen, straight chain, branched chain, or cyclic alkyl of one to about six carbon atoms, $R_6$ and $R_7$ are independently straight chain, branched chain, or cyclic alkyl of one to six carbon atoms, or $R_6$ and $R_7$ together form a straight chain or branched chain alkylene or alkenylene group with four or five carbons in the main alkylene or alkenylene chain, or $R_6$ and $R_7$ together form a group of the formula -A-O-B- or

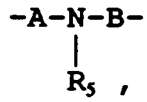 ,

wherein A, B and $R_5$ are as defined hereinabove, or $R_4$ and $R_6$ together form a straight chain or branched chain alkylene group having three or four carbon atoms in the main alkylene chain, with the proviso that when $R_4$ and $R_6$ together form a straight chain or branched chain alkylene group with three or four carbon atoms in the main alkylene chain then $R_7$ is straight chain, branched chain, or cyclic alkyl of one to about six carbon atoms; or (B) of the formula

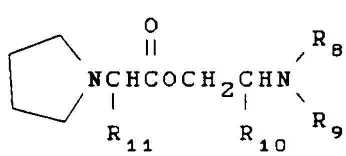

wherein

$R_8$ and $R_9$ are independently straight chain, branched chain, or cyclic alkyl of one to six carbon atoms, or $R_8$ and $R_9$ together form a straight chain or branched chain alkylene group having four or five carbons in the main alkylene chain, or $R_8$ and $R_9$ together form a group of the formula -A-O-B- or

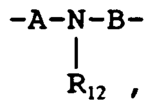

wherein A and B are independently straight chain or branched chain alkylene groups each having two carbon atoms in their main alkylene chain and $R_{12}$ is alkyl, aryl, or a deactivating substituent;
$R_{10}$ is hydrogen, straight chain, branched chain, or cyclic alkyl of one to six carbon atoms, or $R_8$ and $R_{10}$ together form a straight chain or branched chain alkylene group having three or four carbon atoms in the main alkylene chain, with the proviso that when $R_8$ and $R_{10}$ together form a straight chain or branched chain alkylene group having three or four carbon atoms in the main alkylene chain, then $R_9$ is straight chain, branched chain, or cyclic alkyl of one to six carbon atoms;
and $R_{11}$ is hydrogen or straight chain, branched chain, or cyclic alkyl of one to six carbon atoms.

**2.** A curable composition according to Claim 1, wherein the isocyanate-functional material is an isocyanate-functional prepolymer based on an aromatic isocyanate.

**3.** A curable composition according to Claim 1, wherein the compound is selected from: 2-(N,N-dimethylamino)ethyl 1-methylpipecolinate; 2-(1-methylpiperidyl)methyl 1-methylpipecolinate; 2-(1-piperidino)ethyl 1-piperidinoacetate; 2-(1-methylpiperidyl)methyl N,N-dimethylaminoacetate; 2-(1-morpholino)ethyl N,N-diethylaminoacetate; 2-(1-morpholino)ethyl alpha-(N-morpholino)propionate; 2-(N,N-dimethylamino)ethyl N,N-dimethylaminoacetate; 2-(1-pyrrolidino)ethyl 1-methylpipecolinate; 2-(1-piperidino)ethyl N,N-dimethylaminoacetate; 2-(1-piperidino)ethyl 1-pyrrolidineacetate; 2-(4-morpholino)-ethyl 1-pyrrolidineacetate; 2-(N,N-diethylamino)ethyl 1-pyrrolidineacetate; 2-(N,N-dimethylamino)ethyl 1-pyrrolidineacetate; and 2-(1-methylpiperidyl)methyl 1-pyrrolidineacetate.

**4.** A catalysis method for catalyzing the cure of an isocyanate-functional material comprising forming a mixture of:
a) an isocyanate-functional material,
b) water, and
c) a catalytically effective amount of a compound: (A) of the formula

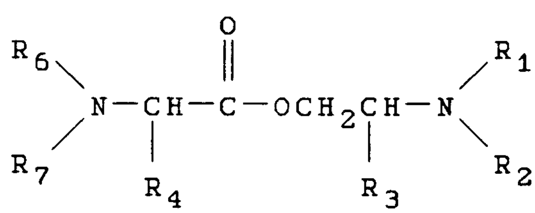

wherein
$R_1$ and $R_2$ are independently straight chain, branched chain, or cyclic alkyl of one to about six carbon atoms, or $R_1$ and $R_2$ together form a straight chain or branched chain alkylene group having four or five carbons in the main alkylene chain, or $R_1$ and $R_2$ together form a group of the formula
-A-O-B- or

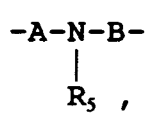

wherein A and B are independently straight chain or branched chain alkylene groups each having two carbon atoms in their main alkylene chain and $R_5$ is alkyl, aryl, or a deactivating substituent;
$R_3$ is hydrogen or straight chain, branched chain, or cyclic alkyl of one to six carbon atoms, or $R_1$ and $R_3$ together form a straight chain or branched chain alkylene group having three or four carbon atoms in the main alkylene chain, with the proviso that when $R_1$ and $R_3$ together form a straight chain or branched chain alkylene group having three or four carbon atoms in the main alkylene chain, then $R_2$ is

straight chain, branched chain, or cyclic alkyl of one to six carbon atoms;
$R_4$ is hydrogen, straight chain, branched chain, or cyclic alkyl of one to six carbon atoms, $R_6$ and $R_7$ are independently straight chain, branched chain, or cyclic alkyl of one to about six carbon atoms, or $R_6$ and $R_7$ together form a straight chain or branched chain alkylene or alkenylene group with four or five carbons in the main alkylene or alkenylene chain, or $R_6$ and $R_7$ together form a group of the formula
-A-O-B- or

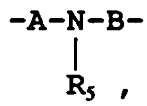

wherein A, B and $R_5$ are as defined hereinabove, or $R_4$ and $R_6$ together form a straight chain or branched chain alkylene group having three or four carbon atoms in the main alkylene chain, with the proviso that when $R_4$ and $R_6$ together form a straight chain or branched chain alkylene group with three or four carbon atoms in the main alkylene chain then $R_7$ is straight chain, branched chain, or cyclic alkyl of one to about six carbon atoms; or (B) of the Formula

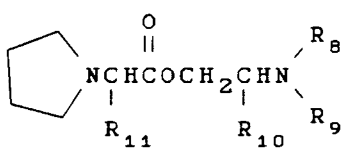

wherein
$R_8$ and $R_9$ are independently straight chain, branched chain, or cyclic alkyl of one to six carbon atoms, or $R_8$ and $R_9$ together form a straight chain or branched chain alkylene group having four or five carbons in the main alkylene chain, or $R_8$ and $R_9$ together form a group of the formula
-A-O-B- or

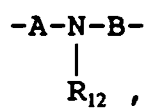

wherein A and B are independently straight chain or branched chain alkylene groups each having two carbon atoms in their main alkylene chain and $R_{12}$ is alkyl, aryl, or a deactivating substituent;
$R_{10}$ is hydrogen, straight chain, branched chain, or cyclic alkyl of one to six carbon atoms, or $R_8$ and $R_{10}$ together form a straight chain or branched chain alkylene group having three or four carbon atoms in the main alkylene chain, with the proviso that when $R_8$ and $R_{10}$ together form a straight chain or branched chain alkylene group having three or four carbon atoms in the main alkylene chain, then $R_9$ is straight chain, branched chain, or cyclic alkyl of one to six carbon atoms;
and $R_{11}$ is hydrogen or straight chain, branched chain, or cyclic alkyl of one to six carbon atoms.

5. A method according to claim 4, wherein the isocyanate-functional material is an isocyanate-functional prepolymer based on an aromatic isocyanate.

6. A method according to Claim 4, wherein the compound is selected from: 2-(N,N-dimethylamino)ethyl 1-methylpipecolinate; 2-(1-methylpiperidyl)methyl 1-methylpipecolinate; 2-(1-piperidino)ethyl 1-piperidinoacetate; 2-(1-methylpiperidyl)methyl N,N-dimethylaminoacetate; 2-(1-morpholino)ethyl N,N-diethylaminoacetate; 2-(1-morpholino)ethyl alpha-(N-morpholino)propionate; 2-(N,N-dimethylamino)ethyl N,N-dimethylaminoacetate; 2-(1-pyrrolidino)ethyl 1-methylpipecolinate; 2-(1-piperidino)ethyl N,N-dimethylaminoacetate; 2-(1-piperidino)ethyl 1-pyrrolidineacetate; 2-(4-morpholino)ethyl 1-pyrrolidineacetate; 2-(N,N-diethylamino)ethyl 1-pyrrolidineacetate; 2-(N,N-dimethylamino)ethyl 1-pyrrolidineacetate; and 2-(1-methylpiperidyl)methyl 1-pyrrolidineacetate.

**7.** An article comprising a flexible sheet material coated with a composition according to Claim 1.

**8.** An article according to Claim 7 in the form of an orthopedic casting tape.

**9.** A method of orthopedic casting comprising the steps of:
(i) providing a flexible sheet material coated with a composition according to Claim 1;
(ii) contacting the coated sheet material with water in order to initiate cure of the composition; and
(iii) applying the coated sheet material to a body member of a subject.

**10.** A compound of the formula

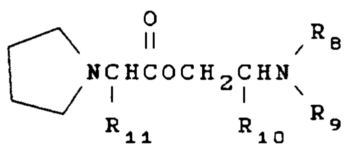

wherein
$R_8$ and $R_9$ are independently straight chain, branched chain, or cyclic alkyl of one to six carbon atoms, or $R_8$ and $R_9$ together form a straight chain or branched chain alkylene group having four or five carbons in the main alkylene chain, or $R_8$ and $R_9$ together form a group of the formula
-A-O-B- or

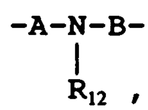
,

wherein A and B are independently straight chain or branched chain alkylene groups each having two carbon atoms in their main alkylene chain and $R_{12}$ is alkyl, aryl, or a deactivating substituent;
$R_{10}$ is hydrogen, straight chain, branched chain, or cyclic alkyl of one to six carbon atoms, or $R_8$ and $R_{10}$ together form a straight chain or branched chain alkylene group having three or four carbon atoms in the main alkylene chain, with the proviso that when $R_8$ and $R_{10}$ together form a straight chain or branched chain alkylene group having three or four carbon atoms in the main alkylene chain, then $R_9$ is straight chain, branched chain, or cyclic alkyl of one to six carbon atoms;
and $R_{11}$ is hydrogen or straight chain, branched chain, or cyclic alkyl of one to six carbon atoms.

## Patentansprüche

**1.** Härtbare Zusammensetzung, umfassend (i) ein Isocyanatfunktionelles Material und (ii) eine katalytisch wirksame Menge einer Verbindung: (A) der Formel

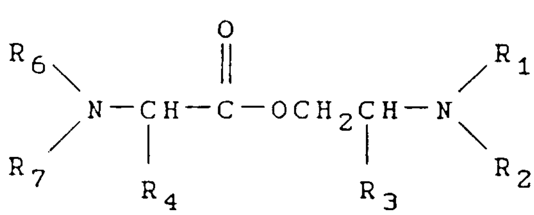

worin
$R_1$ und $R_2$ unabhängig voneinander geradkettiges, verzweigtkettiges oder cyclisches Alkyl mit einem bis sechs Kohlenstoffatomen sind, oder $R_1$ und $R_2$ zusammen eine geradkettige oder verzweigtkettige Alkylengruppe bilden, die vier oder fünf Kohlenstoffatome in der Haupt-Alkylenkette aufweist, oder $R_1$

und $R_2$ zusammen eine Gruppe der Formel
-A-O-B- oder

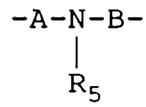

bilden, worin A und B unabhängig voneinander geradkettige oder verzweigtkettige Alkylengruppen sind, wobei jede zwei Kohlenstoffatome in ihrer Haupt-Alkylenkette hat, und $R_5$ Alkyl, Aryl oder ein desaktivierender Substituent ist;
$R_3$ Wasserstoff oder geradkettiges, verzweigtkettiges oder cyclisches Alkyl mit einem bis sechs Kohlenstoffatomen ist, oder $R_1$ und $R_3$ zusammen eine geradkettige oder verzweigtkettige Alkylengruppe bildet, die drei oder vier Kohlenstoffatome in der Haupt-Alkylenkette hat, mit der Maßgabe, daß, wenn $R_1$ und $R_3$ zusammen eine geradkettige oder verzweigtkettige Alkylengruppe bilden, die drei oder vier Kohlenstoffatome in der Haupt-Alkylenkette hat, dann $R_2$ geradkettiges, verzweigtkettiges oder cyclisches Alkyl mit einem bis sechs Kohlenstoffatomen ist;
$R_4$ Wasserstoff, geradkettiges, verzweigtkettiges oder cyclisches Alkyl mit einem bis sechs Kohlenstoffatomen ist, $R_6$ und $R_7$ unabhängig voneinander geradkettiges, verzweigtkettiges oder cyclisches Alkyl mit einem bis sechs Kohlenstoffatomen ist, oder $R_6$ und $R_7$ zusammen eine geradkettige oder verzweigtkettige Alkylen- oder Alkenylengruppe bilden, die vier oder fünf Kohlenstoffatome in der Haupt-Alkylenkette oder Haupt-Alkenylenkette hat, oder $R_6$ und $R_7$ zusammen eine Gruppe der Formel
-A-O-B- oder

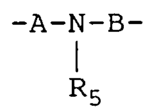

bilden, worin A und B wie oben definiert sind, oder $R_4$ und $R_6$ zusammen eine geradkettige oder verzweigtkettige Alkylengruppe bilden, die drei oder vier Kohlenstoffatome in der Haupt-Alkylenkette hat, mit der Maßgabe, daß, wenn $R_4$ und $R_6$ zusammen eine geradkettige oder verzweigtkettige Alkylengruppe mit drei oder vier Kohlenstoffatomen in der Haupt-Alkylenkette bilden, dann $R_7$ geradkettiges, verzweigtkettiges oder cyclisches Alkyl mit einem bis sechs Kohlenstoffatomen ist;
oder (B) der Formel

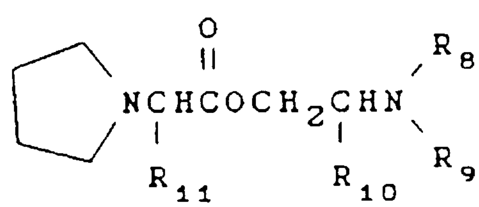

worin
$R_8$ und $R_9$ unabhängig voneinander geradkettiges, verzweigtkettiges oder cyclisches Alkyl mit einem bis sechs Kohlenstoffatomen sind, oder $R_8$ und $R_9$ zusammen eine geradkettige oder verzweigtkettige Alkylengruppe bilden, die vier oder fünf Kohlenstoffatome in der Haupt-Alkylenkette aufweist, oder $R_8$ und $R_9$ zusammen eine Gruppe der Formel
-A-O-B- oder

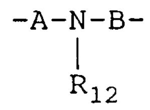

bilden, worin A und B unabhängig voneinander geradkettige oder verzweigtkettige Alkylengruppen sind, wobei jede zwei Kohlenstoffatome in ihrer Haupt-Alkylenkette hat, und $R_{12}$ Alkyl, Aryl oder ein desaktivierender Substituent ist;

$R_{10}$ Wasserstoff, geradkettiges, verzweigtkettiges oder cyclisches Alkyl mit einem bis sechs Kohlenstoffatomen ist, oder $R_8$ und $R_{10}$ zusammen eine geradkettige oder verzweigtkettige Alkylengruppe bilden, die drei oder vier Kohlenstoffatome in der Haupt-Alkylenkette hat, mit der Maßgabe, daß wenn $R_8$ und $R_{10}$ zusammen eine geradkettige oder verzweigtkettige Alkylengruppe bilden, die drei oder vier Kohlenstoffatome in der Haupt-Alkylenkette hat, dann $R_9$ geradkettiges, verzweigtkettiges oder cyclisches Alkyl mit einem bis sechs Kohlenstoffatomen ist; und

$R_{11}$ Wasserstoff oder geradkettiges, verzweigtkettiges oder cyclisches Alkyl mit einem bis sechs Kohlenstoffatomen ist.

**2.** Härtbare Zusammensetzung gemäß Anspruch 1, worin das Isocyanat-funkionelle Material ein Isocyanat-funkionelles Prepolymer basierend auf einem aromatischen Isocyanat ist.

**3.** Härtbare Zusammensetzung gemäß Anspruch 1, worin die Verbindung ausgewählt ist aus:
2-(N,N-Dimethylamino)ethyl-1-methylpipecolinat;
2-(1-Methylpiperidyl)methyl-1-methylpipecolinat;
2-(1-Piperidino)ethyl-1-piperidinoacetat;
2-(1-Methylpiperidyl)methyl-N,N-dimethylaminoacetat;
2-(1-Morpholino)ethyl-N,N-diethylaminoacetat;
2-(1-Morpholino)ethyl-α-(N-morpholino)propionat;
2-(N,N-Dimethylamino)ethyl-N,N-dimethylaminoacetat;
2-(1-Pyrrolidino)ethyl-1-methylpipecolinat;
2-(1-Piperidino)ethyl-N,N-dimethylaminoacetat;
2-(1-Piperidino)ethyl-1-pyrrolidinacetat;
2-(4-Morpholino)ethyl-1-pyrrolidinacetat;
2-(N,N-Diethylamino)ethyl-1-pyrrolidinacetat;
2-(N,N-Dimethylamino)ethyl-1-pyrrolidinacetat und
2-(1-Methylpiperidyl)methyl-1-pyrrolidinacetat.

**4.** Katalyse-Verfahren zum Katalysieren der Härtung eines Isocyanat-funktionellen Materials, umfassend die Bildung einer Mischung aus:
a) einem Isocyanat-funktionellen Material
b) Wasser und
c) einer katalytisch wirksamen Menge einer Verbindung:
(A) der Formel

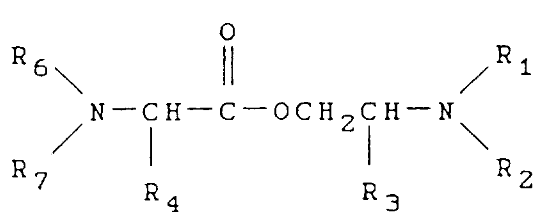

worin
$R_1$ und $R_2$ unabhängig voneinander geradkettiges, verzweigtkettiges oder cyclisches Alkyl mit einem bis sechs Kohlenstoffatomen sind, oder $R_1$ und $R_2$ zusammen eine geradkettige oder verzweigtkettige Alkylengruppe bilden, die vier oder fünf Kohlenstoffatome in der Haupt-Alkylenkette aufweist, oder $R_1$

und $R_2$ zusammen eine Gruppe der Formel

-A-O-B- oder

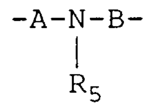

bilden, worin A und B unabhängig voneinander geradkettige oder verzweigtkettige Alkylengruppen sind, wobei jede zwei Kohlenstoffatome in ihrer Haupt-Alkylenkette hat, und $R_5$ Alkyl, Aryl oder ein desaktivierender Substituent ist;

$R_3$ Wasserstoff oder geradkettiges, verzweigtkettiges oder cyclisches Alkyl mit einem bis sechs Kohlenstoffatomen ist, oder $R_1$ und $R_3$ zusammen eine geradkettige oder verzweigtkettige Alkylengruppe bilden, die drei oder vier Kohlenstoffatome in der Haupt-Alkylenkette hat, mit der Maßgabe, daß, wenn $R_1$ und $R_3$ zusammen eine geradkettige oder verzweigtkettige Alkylengruppe bilden, die drei oder vier Kohlenstoffatome in der Haupt-Alkylenkette hat, dann $R_2$ geradkettiges, verzweigtkettiges oder cyclisches Alkyl mit einem bis sechs Kohlenstoffatomen ist;

$R_4$ Wasserstoff, geradkettiges, verzweigtkettiges oder cyclisches Alkyl mit einem bis sechs Kohlenstoffatomen ist, $R_6$ und $R_7$ unabhängig voneinander geradkettiges, verzweigtkettiges oder cyclisches Alkyl mit einem bis sechs Kohlenstoffatomen sind, oder $R_6$ und $R_7$ zusammen eine geradkettige oder verzweigtkettige Alkylen- oder Alkenylengruppe bilden, die vier oder fünf Kohlenstoffatome in der Haupt-Alkylenkette oder Haupt-Alkenylengruppe hat, oder $R_6$ und $R_7$ zusammen eine Gruppe der Formel

-A-O-B- oder

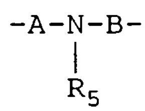

bilden, worin A und B und $R_5$ wie oben definiert sind, oder $R_4$ und $R_6$ zusammen eine geradkettige oder verzweigtkettige Alkylengruppe bilden, die drei oder vier Kohlenstoffatome in der Haupt-Alkylenkette hat, mit der Maßgabe, daß wenn $R_4$ und $R_6$ zusammen eine geradkettige oder verzweigtkettige Alkylengruppe drei oder vier Kohlenstoffatomen in der Haupt-Alkylenkette bilden, dann $R_7$ geradkettiges, verzweigtkettiges oder cyclisches Alkyl mit einem bis sechs Kohlenstoffatomen ist;

oder (B) der Formel

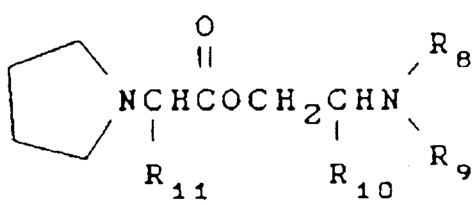

worin

$R_8$ und $R_9$ unabhängig voneinander geradkettiges, verzweigtkettiges oder cyclisches Alkyl mit einem bis sechs Kohlenstoffatomen sind, oder $R_8$ und $R_9$ zusammen eine geradkettige oder verzweigtkettige Alkylengruppe bilden, die vier oder fünf Kohlenstoffatome in der Haupt-Alkylenkette aufweist, oder $R_8$ und $R_9$ zusammen eine Gruppe der Formel

-A-O-B- oder

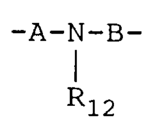

bilden, worin A und B unabhängig voneinander geradkettige oder verzweigtkettige Alkylengruppen sind, wobei jede zwei Kohlenstoffatome in ihrer Haupt-Alkylenkette hat, und $R_{12}$ Alkyl, Aryl oder ein desaktivierender Substituent ist;

$R_{10}$ Wasserstoff, geradkettiges, verzweigtkettiges oder cyclisches Alkyl mit einem bis sechs Kohlenstoffatomen ist, oder $R_8$ und $R_{10}$ zusammen eine geradkettige oder verzweigtkettige Alkylengruppe bilden, die drei oder vier Kohlenstoffatome in der Haupt-Alkylenkette hat, mit der Maßgabe, daß, wenn $R_8$ und $R_{10}$ zusammen eine geradkettige oder verzweigtkettige Alkylengruppe bilden, die drei oder vier Kohlenstoffatome in der Haupt-Alkylenkette hat, dann $R_9$ geradkettiges, verzweigtkettiges oder cyclisches Alkyl mit einem bis sechs Kohlenstoffatomen ist; und

$R_{11}$ Wasserstoff oder geradkettiges, verzweigtkettiges oder cyclisches Alkyl mit einem bis sechs Kohlenstoffatomen ist.

**5.** Verfahren gemäß Anspruch 4, worin das Isocyanat-funktionelle Material ein Isocyanat-funktionelles Prepolymer basierend auf einem aromatischen Isocyanat ist.

**6.** Verfahren gemäß Anspruch 4, worin die Verbindung ausgewählt ist aus:
2-(N,N-Dimethylamino)ethyl-1-methylpipecolinat;
2-(1-Methylpiperidyl)methyl-1-methylpipecolinat;
2-(1-Piperidino)ethyl-1-piperidinoacetat;
2-(1-Methylpiperidyl)methyl-N,N-dimethylaminoacetat;
2-(1-Morpholino)ethyl-N,N-diethylaminoacetat;
2-(1-Morpholino)ethyl-α-(N-morpholino)propionat;
2-(N,N-Dimethylamino)ethyl-N,N-dimethylaminoacetat;
2-(1-Pyrrolidino)ethyl-1-methylpipecolinat;
2-(1-Piperidino)ethyl-N,N-dimethylaminoacetat;
2-(1-Piperidino)ethyl-1-pyrrolidinacetat;
2-(4-Morpholino)ethyl-1-pyrrolidinacetat;
2-(N,N-Diethylamino)ethyl-1-pyrrolidinacetat;
2-(N,N-Dimethylamino)ethyl-1-pyrrolidinacetat und
2-(1-Methylpiperidyl)methyl-1-pyrrolidinacetat.

**7.** Gegenstand, umfassend ein flexibles Blatt-Material, das mit einer Zusammensetzung gemaß Anspruch 1 beschichtet ist.

**8.** Gegenstand gemäß Anspruch 7 in Form eines Gießbandes für orthopädische Zwecke.

**9.** Gießverfahren für orthopädische Zwecke, umfassend die Stufen:
(i) Bereitstellen eines flexiblen Blatt-Materials, das mit einer Zusammensetzung gemäß Anspruch 1 beschichtet ist;
(ii) In-Kontakt-Bringen des beschichteten Blatt-Materials mit Wasser, um die Härtung der Zusammensetzung zu initiieren, und
(iii) Aufbringen des beschichteten Blatt-Materials auf einen Körperteil einer Person.

**10.** Verbindung der Formel

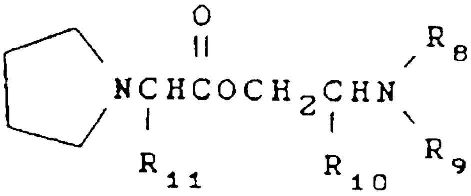

worin

$R_8$ und $R_9$ unabhängig voneinander geradkettiges, verzweigtkettiges oder cyclisches Alkyl mit einem bis sechs Kohlenstoffatomen sind, oder $R_8$ und $R_9$ zusammen eine geradkettige oder verzweigtkettige Alkylengruppe bilden, die vier oder fünf Kohlenstoffatome in der Haupt-Alkylenkette aufweist, oder $R_8$

und $R_9$ zusammen eine Gruppe der Formel

-A-O-B- oder

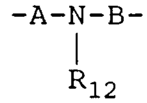

bilden, worin A und B unabhängig voneinander geradkettige oder verzweigtkettige Alkylengruppen sind, wobei jede zwei Kohlenstoffatome in ihrer Haupt-Alkylenkette hat, und $R_{12}$ Alkyl, Aryl oder ein desaktivierender Substituent ist;

$R_{10}$ Wasserstoff, geradkettiges, verzweigtkettiges oder cyclisches Alkyl mit einem bis sechs Kohlenstoffatomen ist, oder $R_8$ und $R_{10}$ zusammen eine geradkettige oder verzweigtkettige Alkylengruppe bilden, die drei oder vier Kohlenstoffatome in der Haupt-Alkylenkette hat, mit der Maßgabe, daß, wenn $R_8$ und $R_{10}$ zusammen eine geradkettige oder verzweigtkettige Alkylengruppe bilden, die drei oder vier Kohlenstoffatome in der Haupt-Alkylenkette hat, dann $R_9$ geradkettiges, verzweigtkettiges oder cyclisches Alkyl mit einem bis sechs Kohlenstoffatomen ist; und

$R_{11}$ Wasserstoff oder geradkettiges, verzweigtkettiges oder cyclisches Alkyl mit einem bis sechs Kohlenstoffatomen ist.

**Revendications**

**1.** Composition durcissable comprenant (i) une matière à fonction isocyanate ; et (ii) une quantité catalytiquement efficace d'un composé : (A) de formule

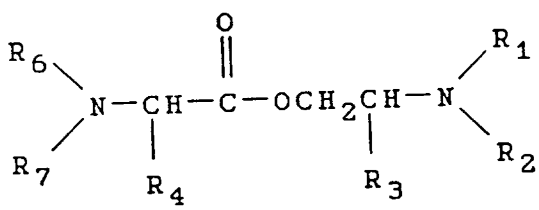

dans laquelle $R_1$ et $R_2$ représentent, indépendamment, un groupe alkyle à chaîne droite, à chaîne ramifiée ou cyclique ayant 1 à 6 atomes de carbone, ou bien $R_1$ et $R_2$ forment ensemble un groupe alkylène à chaîne droite ou à chaîne ramifiée ayant 4 ou 5 atomes de carbone dans la chaîne alkylénique principale, ou bien $R_1$ et $R_2$ forment ensemble un groupe de formule

-A-O-B- ou

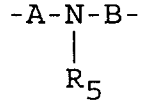

dans laquelle A et B représentent indépendamment des groupes alkylène à chaîne droite ou à chaîne ramifiée ayant chacun deux atomes de carbone dans leur chaîne alkylénique principale et $R_5$ est un groupe alkyle, aryle ou un substituant désactivant ;

$R_3$ est de l'hydrogène ou un groupe alkyle à chaîne droite, à chaîne ramifiée ou cyclique ayant 1 à 6 atomes de carbone, ou bien $R_1$ et $R_3$ forment ensemble un groupe alkylène à chaîne droite ou à chaîne ramifiée ayant 3 ou 4 atomes de carbone dans la chaîne alkylénique principale, sous réserve que lorsque $R_1$ et $R_3$ forment ensemble un groupe alkylène à chaîne droite ou à chaîne ramifiée ayant 3 ou 4 atomes de carbone dans la chaîne alkylénique principale, $R_2$ soit alors un groupe alkyle à chaîne droite, à chaîne ramifiée ou cyclique ayant 1 à environ 6 atomes de carbone ;

$R_4$ est de l'hydrogène, un groupe alkyle à chaîne droite, à chaîne ramifiée ou cyclique ayant 1 à environ 6 atomes de carbone, $R_6$ et $R_7$ représentent indépendamment un groupe alkyle à chaîne

droite, à chaîne ramifiée ou cyclique ayant 1 à 6 atomes de carbone, ou bien $R_6$ et $R_7$ forment ensemble un groupe alkylène ou alcénylène à chaîne droite ou à chaîne ramifiée ayant 4 ou 5 atomes de carbone dans la chaîne alkylénique ou alcénylénique principale, ou bien $R_6$ et $R_7$ forment ensemble un groupe de formule -A-O-B-ou

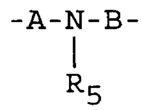

dans laquelle A, B et $R_5$ sont tels que définis ci-dessus, ou bien $R_4$ et $R_6$ forment ensemble un groupe alkylène à chaîne droite ou à chaîne ramifiée ayant 3 ou 4 atomes de carbone dans la chaîne alkylénique principale sous réserve que lorsque $R_4$ et $R_6$ forment ensemble un groupe alkylène à chaîne droite ou à chaîne ramifiée ayant 3 ou 4 atomes de carbone dans la chaîne alkylénique principale, $R_7$ soit alors un groupe alkyle à chaîne droite, à chaîne ramifiée ou cyclique ayant 1 à environ 6 atomes de carbone ; ou bien (B) de formule

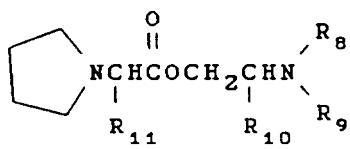

dans laquelle

$R_8$ et $R_9$ représentent indépendamment un groupe alkyle à chaîne droite, à chaîne ramifiée ou cyclique ayant 1 à 6 atomes de carbone, ou bien $R_8$ et $R_9$ forment ensemble un groupe alkylène à chaîne droite ou à chaîne ramifiée ayant 4 ou 5 atomes de carbone dans la chaîne alkylénique principale, ou bien $R_8$ et $R_9$ forment ensemble un groupe de formule -A-O-B- ou

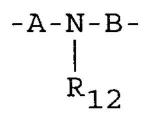

dans laquelle A et B représentent indépendamment des groupes alkylène à chaîne droite ou à chaîne ramifiée ayant chacun deux atomes de carbone dans leur chaîne alkylénique principale et $R_{12}$ est un groupe alkyle, aryle ou un substituant désactivant ;

$R_{10}$ est de l'hydrogène, un groupe alkyle à chaîne droite, à chaîne ramifiée ou cyclique ayant 1 à 6 atomes de carbone, ou bien $R_8$ et $R_{10}$ forment ensemble un groupe alkylène à chaîne droite ou à chaîne ramifiée ayant 3 ou 4 atomes de carbone dans la chaîne alkylénique principale, sous réserve que lorsque $R_8$ et $R_{10}$ forment ensemble un groupe alkylène à chaîne droite ou à chaîne ramifiée ayant 3 ou 4 atomes de carbone dans la chaîne alkylénique principale, $R_9$ soit alors un groupe alkyle à chaîne droite, à chaîne ramifiée ou cyclique ayant 1 à 6 atomes de carbone ;

et $R_{11}$ est de l'hydrogène ou un groupe alkyle à chaîne droite, à chaîne ramifiée ou cyclique ayant 1 à 6 atomes de carbone.

**2.** Composition durcissable suivant la revendication 1, dans laquelle la matière à fonction isocyanate est un prépolymère à fonction isocyanate à base d'un isocyanate aromatique.

**3.** Composition durcissable suivant la revendication 1, dans laquelle le composé est choisi entre

le 1-méthylpipécolinate de 2-(N,N-diméthylamino)-éthyle ;
le 1-méthylpipécolinate de 2-(1-méthylpipéridyl)-méthyle ;
le 1-pipéridino-acétate de 2-(1-pipéridino)éthyle ;
le N,N-diméthylamino-acétate de 2-(1-méthylpipéridyl)méthyle ;
le N,N-diéthylamino-acétate de 2-(1-morpholino)-éthyle ;
l'alpha-(N-morpholino)propionate de 2-(1-morpholino)éthyle,

le N,N-diméthylamino-acétate de 2-(N,N-diméthylamino)éthyle ;
le 1-méthylpipécolinate de 2-(1-pyrrolidino)é-thyle ;
le N,N-diméthylamino-acétate de 2-(1-pipéridino)-éthyle ;
le 1-pyrrolidine-acétate de 2-(1-pipéridino)-éthyle ;
le 1-pyrrolidine-acétate de 2-(4-morpholino)-éthyle ;
le 1-pyrrolidine-acétate de 2-(N,N-diéthylamino)-éthyle ;
le 1-pyrrolidine-acétate de 2-(N,N-diméthylamino)éthyle et
le 1-pyrrolidine-acétate de 2-(1-méthylpipéridyl)méthyle.

**4.** Procédé pour la catalyse du durcissement d'une matière à fonction isocyanate, comprenant la formation d'un mélange :

a) d'une matière à fonction isocyanate ;
b) d'eau, et
c) d'une quantité catalytiquement efficace d'un composé (A) de formule

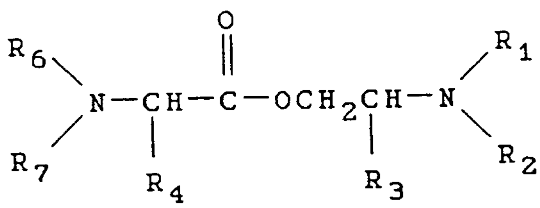

dans laquelle

$R_1$ et $R_2$ représentent, indépendamment, un groupe alkyle à chaîne droite, à chaîne ramifiée ou cyclique ayant 1 à 6 atomes de carbone, ou bien $R_1$ et $R_2$ forment ensemble un groupe alkylène à chaîne droite ou à chaîne ramifiée ayant 4 ou 5 atomes de carbone dans la chaîne alkylénique principale, ou bien $R_1$ et $R_2$ forment ensemble un groupe de formule

-A-O-B- ou

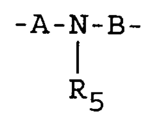

dans laquelle A et B représentent indépendamment des groupes alkylène à chaîne droite ou à chaîne ramifiée ayant chacun deux atomes de carbone dans leur chaîne alkylénique principale et $R_5$ est un groupe alkyle, aryle ou un substituant désactivant ;

$R_3$ est de l'hydrogène ou un groupe alkyle à chaîne droite, à chaîne ramifiée ou cyclique ayant 1 à 6 atomes de carbone, ou bien $R_1$ et $R_3$ forment ensemble un groupe alkylène à chaîne droite ou à chaîne ramifiée ayant 3 ou 4 atomes de carbone dans la chaîne alkylénique principale, sous réserve que lorsque $R_1$ et $R_3$ forment ensemble un groupe alkylène à chaîne droite ou à chaîne ramifiée ayant 3 ou 4 atomes de carbone dans la chaîne alkylénique principale, $R_2$ soit alors un groupe alkyle à chaîne droite, à chaîne ramifiée ou cyclique ayant 1 à 6 atomes de carbone ;

$R_4$ est de l'hydrogène, un groupe alkyle à chaîne droite, à chaîne ramifiée ou cyclique ayant 1 à 6 atomes de carbone, $R_6$ et $R_7$ représentent indépendamment un groupe alkyle à chaîne droite, à chaîne ramifiée ou cy-clique ayant 1 à 6 atomes de carbone, ou bien $R_6$ et $R_7$ forment ensemble un groupe alkylène ou alcénylène à chaîne droite ou à chaîne ramifiée ayant 4 ou 5 atomes de carbone dans la chaîne alkylénique ou alcénylénique principale, ou bien $R_6$ et $R_7$ forment ensemble un groupe de formule -A-O-B-ou

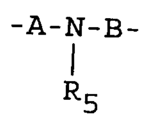

dans laquelle A, B et $R_5$ sont tels que définis ci-dessus, ou bien $R_4$ et $R_6$ forment ensemble un groupe alkylène à chaîne droite ou à chaîne ramifiée ayant 3 ou 4 atomes de carbone dans la chaîne alkylénique principale sous réserve que lorsque $R_4$ et $R_6$ forment ensemble un groupe alkylène à chaîne droite ou à chaîne ramifiée ayant 3 ou 4 atomes de carbone dans la chaîne alkylénique principale, $R_7$ soit alors un groupe alkyle à chaîne droite, à chaîne ramifiée ou cyclique ayant 1 à environ 6 atomes de carbone ; ou bien (B) de formule

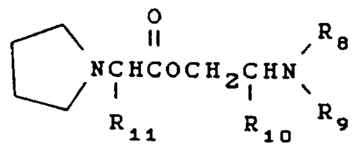

dans laquelle

$R_8$ et $R_9$ représentent indépendamment un groupe alkyle à chaîne droite, à chaîne ramifiée ou cyclique ayant 1 à 6 atomes de carbone, ou bien $R_8$ et $R_9$ forment ensemble un groupe alkylène à chaîne droite ou à chaîne ramifiée ayant 4 ou 5 atomes de carbone dans la chaîne alkylénique principale, ou bien $R_8$ et $R_9$ forment ensemble un groupe de formule -A-O-B- ou

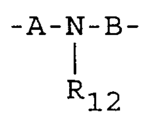

dans laquelle A et B représentent indépendamment des groupes alkylène à chaîne droite ou à chaîne ramifiée ayant chacun deux atomes de carbone dans leur chaîne alkylénique principale et $R_{12}$ est un groupe alkyle, aryle ou un substituant désactivant ;

$R_{10}$ est de l'hydrogène, un groupe alkyle à chaîne droite, à chaîne ramifiée ou cyclique ayant 1 à 6 atomes de carbone, ou bien $R_8$ et $R_{10}$ forment ensemble un groupe alkylène à chaîne droite ou à chaîne ramifiée ayant 3 ou 4 atomes de carbone dans la chaîne alkylénique principale, sous réserve que lorsque $R_8$ et $R_{10}$ forment ensemble un groupe alkylène à chaîne droite ou à chaîne ramifiée ayant 3 ou 4 atomes de carbone dans la chaîne alkylénique principale, $R_9$ soit alors un groupe alkyle à chaîne droite, à chaîne ramifiée ou cyclique ayant 1 à 6 atomes de carbone ;

et $R_{11}$ est de l'hydrogène ou un groupe alkyle à chaîne droite, à chaîne ramifiée ou cyclique ayant 1 à 6 atomes de carbone.

**5.** Procédé suivant la revendication 4, dans lequel la matière à fonction isocyanate est un prépolymère à fonction isocyanate à base d'un isocyanate aromatique.

**6.** Procédé suivant la revendication 4, dans lequel le composé est choisi entre :
le 1-méthylpipécolinate de 2-(N,N-diméthylamino)-éthyle ;
le 1-méthylpipécolinate de 2-(1-méthylpipéridyl)-méthyle ;
le 1-pipéridino-acétate de 2-(1-pipéridino)éthyle ;
le N,N-diméthylamino-acétate de 2-(1-méthylpipéridyl)méthyle ;
le N,N-diéthylamino-acétate de 2-(1-morpholino)-éthyle ;
l'alpha-(N-morpholino)propionate de 2-(1-morpholino)éthyle,
le N,N-diméthylamino-acétate de 2-(N,N-diméthylamino)éthyle ;
le 1-méthylpipécolinate de 2-(1-pyrrolidino)-éthyle ;
le N,N-diméthylamino-acétate de 2-(1-pipéridino)-éthyle ;
le 1-pyrrolidine-acétate de 2-(1-pipéridino)-éthyle ;
le 1-pyrrolidine-acétate de 2-(4-morpholino)-éthyle ;
le 1-pyrrolidine-acétate de 2-(N,N-diéthylamino)-éthyle ;
le 1-pyrrolidine-acétate de 2-(N,N-diméthylamino)éthyle et
le 1-pyrrolidine-acétate de 2-(1-méthylpipéridyl)méthyle.

**7.** Article comprenant une matière en feuille flexible revêtue d'une composition suivant la revendication 1.

**8.** Article suivant la revendication 7, sous forme d'une bande de moulage orthopédique.

**9.** Procédé de moulage orthopédique, qui comprend les étapes consistant :

(i) à prendre une matière en feuille flexible revêtue d'une composition suivant la revendication 1 ;

(ii) à mettre la matière en feuille revêtue en contact avec de l'eau pour amorcer le durcissement de la composition ; et

(iii) à appliquer la matière en feuille revêtue sur une partie du corps d'un individu.

**10.** Composé de formule

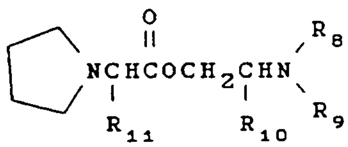

dans laquelle

$R_8$ et $R_9$ représentent indépendamment un groupe alkyle à chaîne droite, à chaîne ramifiée ou cyclique ayant 1 à 6 atomes de carbone, ou bien $R_8$ et $R_9$ forment ensemble un groupe alkylène à chaîne droite ou à chaîne ramifiée ayant 4 ou 5 atomes de carbone dans la chaîne alkylénique principale, ou bien $R_8$ et $R_9$ forment ensemble un groupe de formule -A-O-B- ou

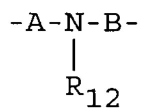

dans laquelle A et B représentent indépendamment des groupes alkylène à chaîne droite ou à chaîne ramifiée ayant chacun deux atomes de carbone dans leur chaîne alkylénique principale et $R_{12}$ est un groupe alkyle, aryle ou un substituant désactivant ;

$R_{10}$ est de l'hydrogène, un groupe alkyle à chaîne droite, à chaîne ramifiée ou cyclique ayant 1 à 6 atomes de carbone ou bien $R_8$ et $R_{10}$ forment ensemble un groupe alkylène à chaîne droite ou à chaîne ramifiée ayant 3 ou 4 atomes de carbone dans la chaîne alkylénique principale, sous réserve que lorsque $R_8$ et $R_{10}$ forment ensemble un groupe alkylène à chaîne droite ou à chaîne ramifiée ayant 3 ou 4 atomes de carbone dans la chaîne alkylénique principale, $R_9$ soit alors un groupe alkyle à chaîne droite, à chaîne ramifiée ou cyclique ayant 1 à 6 atomes de carbone ;

et $R_{11}$ est de l'hydrogène ou un groupe alkyle à chaîne droite, à chaîne ramifiée ou cyclique ayant 1 à 6 atomes de carbone.